# EUROPEAN PATENT APPLICATION

(11) **EP 1 270 031 A1**
(43) Date of publication of application: **02.01.2003**
(21) Application number: 01917486.1
(22) Date of filing: 22.03.2001
(51) Int. Cl.: A61M 5/14, A61M 25/00

(54) **MEDICAL TUBE AND PRODUCTION METHOD AND PRODUCTION DEVICE THEREFOR AND MEDICAL APPLIANCE**

(30) Priority: 22.03.2000 JP 2000079928; 18.10.2000 JP 2000317479; 15.12.2000 JP 2000382696; 15.12.2000 JP 2000382700; 01.02.2001 JP 2001025242
(71) Applicant: Kawasumi Laboratories, Tokyo 140-8555 (JP)
(72) Inventor: Kawano, Takumi, Kawasumi Laboratories, Inc., Tokyo 140-8555 (JP); Ono, Seiichi, Kawasumi Laboratories, Inc., Ono-gun, Oita 879-7153 (JP); Ishida, Akira, Kawasumi Laboratories, Inc., Ono-gun, Oita 879-7153 (JP); Watanabe, Masatoshi, Kawasumi Laboratories, Inc., Ono-gun, Oita 879-7153 (JP)
(74) Representative: Hering, Hartmut, Dipl.-Ing.
(86) International application number: JP0102264
(87) International publication number: WO01070305

(57) **Abstract**

A medical tube having a coil-shaped mono-filament, a small-diameter tubular body, or a bellows-shaped reinforcing member mounted on the outer periphery of the tube, the medical tube being excellent in flexibility and anti-kinking property and capable of greatly saving its material usage. This medical tube is suitably used as constituting members of medical appliances, such as a blood circuit line, a transfusion/infusion set, an infusion bag, and a blood bag.

## Description

### Technical Field

The present invention relates to the improvement in a medical tube, the improved medical tube being capable of greatly saving its material usage and being excellent in flexibility and anti-kinking property. The medical tube of the present invention is preferably used as a constituting member of a medical appliance such as a blood circuit, a transfusion/infusion set, a medical fluid bag, and a blood bag.

### Technical Background

Presently, blood circuits are produced and sold by various makers, and the specifications of 3.2 mm to 4.7 mm in inner diameter and 1.0 mm to 1.3 mm in thickness are adopted for main tubes constituting these blood circuits.

These specifications are determined in consideration of the conditions such as: (1) the priming amount and pressure loss are set according to the constitution, medical treatment conditions, and so on of a patient undergoing dialysis or the like; (2) the main tube is fully resistant against high-pressure steam sterilization (in other words, the tube is not collapsed or blocking is not caused at the time of the high-pressure steam sterilization); and (3) no kink occurs in the tube when in use; and so on.

Conventionally, as materials of such tubes constituting a blood circuit, for example, nonrigid(flexible) polyvinyl chloride has been used, but these tubes are used only once, namely, are disposed after a single use. It has been pointed out that, for some reason, polyvinyl chloride tends to generate toxic substances such as dioxin when it is collected and incinerated after usage.

Accordingly, when resin such as flexible polyvinyl chloride is used, it is recommended to reduce the usage of the resin as much as possible and to recycle it for the purpose of environmental protection and effective utilization of resources.

Further, studies on medical tubes made of a material not containing polyvinyl chloride have also been actively conducted in recent years. For example, polyethylene (PE), ethylene vinyl acetate copolymer (EVA), polybutadiene (PB), and the like are available as materials of the medical tubes not containing polyvinyl chloride, and though styrene-based elastomer is also studied, the use of styrene-based elastomer results in very high material cost compared with polyvinyl chloride. Moreover, it is generally considered that the medical tubes need to have property that satisfies the conditions of having an appropriate degree of flexibility without liability to undergo kinking or being kept bended when the tubes are bent, and also other conditions. The tubes made of the above materials, though having flexibility, have such a drawback of being liable to undergo kinking.

In view of the above, it is required that the amount of resin material in use should be reduced from the viewpoint of preventing cost increase and lowering the generation of toxic substances when any of the above resins is used. And such medical tubes of resin are required whose performance necessary as the medical tube such as anti-kinking property is not impaired even when the amount of resin in use is thus reduced.

Under the circumstances, it is an object of the present invention to provide a medical tube preferably used as a constituting member of a blood circuit line and so on, the medical tube capable of greatly (for example, to a half or less) saving the usage of its constituting material and enhance various functions as a tube.

### Disclosure of the Invention

The present invention is made from the above viewpoints. According to the present invention, the following inventions are provided.
[1] A medical tube, wherein a coil-shaped reinforcing member is mounted on the outer periphery of said tube.
[2] The medical tube according to [1], wherein said reinforcing member is a mono-filament or a small-diameter tubular body.
[3] The medical tube according to [1] , wherein said reinforcing member is a bellows-shaped reinforcing member having a coil-shaped concave-convex.
[4] The medical tube according to [1] or [2], wherein said coil-shaped reinforcing member is so formed that the pitch P is 2.0 to 5.0 mm.
[5] The medical tube according to [1], [2], or [4],
   wherein said coil-shaped reinforcing member is so formed that the number of coils NC on the outer periphery of said tube is 1 to 10.
[6] The medical tube according to [1], [2], [4], or [5], wherein said coil-shaped reinforcing member is so formed that the lead L on the outer periphery of said tube is 2.0 to 50.0 mm.
[7] The medical tube according to any one of [1], [2], and [4] to [6], wherein said coil-shaped reinforcing member is so formed that the winding angle θ relative to a center line C of said tube in a longitudinal direction is 60 degrees to 85 degrees.
[8] The medical tube according to any one of [1], [2], and [4] to [7], wherein the area S that the coil-shaped reinforcing member covering the surface of said tube occupies is 30 % or larger.
[9] The medical tube according to [3], wherein said bellows-shaped reinforcing member is so formed that a pitch P of the protruding portion T is 1.0 to 5.0 mm.
[10] The medical tube according to [3] or [8], wherein the angle θ made by the center line C of said tube in a longitudinal direction and said protruding portion T is 60 degrees to 90 degrees.
[11] A production method for the medical tube according to any one of [1], [2], and [4] to [8], the production method comprising the steps of:
   (1) extruding the tube from an extrusion die; and
   (2) mounting the reinforcing member in the coil form on the outer periphery of the tube while said extruded tube is hot.
[12] A production method for the medical tube according to any one of [1], [2], and [4] to [8], the production method comprising the steps of:
   (1) extruding the tube from an extrusion die;
   (2) extruding the reinforcing member from another extrusion die; and
   (3) mounting said extruded reinforcing member in a coil form on the outer periphery of said extruded tube.
[13] A production device for the medical tube according to any one of [1], [2], and (4) to (8) , the production device comprising an extrusion molding means for the tube, a take-up means for the reinforcing member, a supply means for the reinforcing member, and a rotation applying means for the take-up means,
   wherein said rotation applying means transmits rotation to said take-up means, said supply means for the reinforcing member feeds the reinforcing member, and said take-up means takes up said reinforcing member to send said taken-up reinforcing member in an extruding direction of the tube extruded from the extrusion molding means in sequence, thereby mounting said reinforcing member on the outer periphery of said tube.
[14] The production device for the medical tube according to [13],
   wherein said extruding means for the tube comprises an extrusion die for the tube composed of an outer die and an inner die,
   wherein said take-up means for the reinforcing member is constituted by a take-up device,
   wherein said supply means for the reinforcing member is constituted by a supply device, and
   wherein said rotation applying means for the take-up means is constituted by a motor M.
[15] A production device for the medical tube according to any one of [1], [2], and [4] to [8], the production device comprising an extrusion molding means for the tube, a take-up means for the reinforcing member, an extruding means for the reinforcing member, and a rotation applying means for the take-up means,
   wherein said rotation applying means transmits rotation to said take-up means, and said extruding means for the reinforcing member extrudes the reinforcing member in the extruding direction of the tube extruded from said extrusion molding means, thereby mounting said reinforcing member on the outer periphery of said tube.
[16] The production device for the medical tube according to [15] ,
   wherein said extruding means for the tube comprises an extrusion die for the tube composed of an outer die and an inner die,
   wherein said take-up means for the reinforcing member is constituted by a take-up device,
   wherein said extruding means for the reinforcing member is constituted by an extrusion die, and
   wherein said rotation applying means for the take-up means is constituted by a motor M and a belt.
[17] A production method for the medical tube according to any one of [1], [3], [9] or [10], the production method comprising the steps of:
   (1) extruding a constituting material of the tube in a tube shape;
   (2) extruding a constituting material of the reinforcing member onto the outer periphery of said extruded tube;
   (3) sandwiching outer peripheries of said constituting material of the tube and said constituting material of the reinforcing member by molding dies while both of the constituting materials are extruded at the same time; and
   (4) absorbing spaces between said constituting material of the reinforcing member and said molding dies, bringing said constituting material of the reinforcing member into close contact with molding grooves of the molding dies, and forming the coil-shaped concave-convex in said constituting material of the reinforcing member to give it a bellows-shape.
(18) A production device for the medical tube according to any one of [1], [3], [9] or [10], the production device comprising:
   an extruding device for the tube; an extruding device for extruding the reinforcing member onto the outer periphery of the tube; and a molding device for the extruded reinforcing member.
[19] The production device for the medical tube according to [18],
   wherein said molding device for the reinforcing member comprises a molding die for the reinforcing member, a jig for said molding die, a vacuum pump, and a drive device for said molding die, and
   wherein a pair of said jigs are disposed to face each other, and said molding die is movably provided on an outer periphery of each of said jigs.
[20] The production device for the medical tube according to [19] , wherein said molding die is constituted by integrating a plurality of dies, between each of which a ventilation hole is formed, and having provided a molding groove for said reinforcing member.
(21) The production device for the medical tube according to [19], wherein said jig has grooves formed to communicate with said ventilation holes and ventilation holes formed to communicate with said grooves and said vacuum pump.
[22] A medical tube, wherein a large-diameter connecting tube is mounted on the outer periphery of one end portion of the tube according to any one of [1] to [10] and a small-diameter connecting tube is mounted on said large-diameter connecting tube thereby enabling a connection of a connecting pipe to said small-diameter connecting tube.
[23] A medical tube, wherein a flat portion is formed on the outer periphery of one end portion of the tube according to any one of [1] to [10] to enable a connecting pipe to be mounted on said flat portion.
[24] A medical tube member, wherein a connecting pipe is mounted on one end portion of said tube according to any one of [1] to [10], or [22], or [23].
[25] A medical tube member, wherein a connecting pipe is mounted on one end portion of said tube according to any one of [1] to [10], the connecting pipe having a thread groove matching the shape of the outer periphery of the end portion of said tube.
[26] A medical tube member in which said tube according to any one of claims 1 to 10 and 22 to 23 and a connecting pipe 41B having provided a protruding small-diameter portion 43B are connected to each other,
   wherein said tube is mounted on the outer periphery of said small-diameter portion 43 of the connecting pipe 41B.
[27] A medical tube member in which said tube according to any one of claims 1 to 10 and 22 to 23 and a connecting pipe 41 having a groove portion 45 are connected to each other,
   wherein an outer tubular turn-up portion 34 is formed at the tip of said tube and the outer periphery of said turn-up portion 34 is mounted on said groove portion 45 of the connecting pipe 41.
[28] A medical tube member in which said tube according to any one of claims 1 to 10 and 22 to 23 and a connecting pipe 41 having a groove portion 45 are connected to each other,
   wherein an outer tubular turn-up portion 34 is formed at the tip of the tube 31A on which said reinforcing member is mounted,
   and the outer periphery of said turn-up portion 34 is mounted on said groove portion 45 of the connecting pipe 41, and
   wherein a caulking ring 51 is pressure-inserted into a space between the forward portion of the tube 31 on which said reinforcing member is mounted and said turn-up portion 34 or into a space surrounded by said forward portion of the tube, said turn-up portion 34, and said groove portion 45.
[29] A medical appliance, wherein, in a blood circuit comprising an artery-side circuit and a vein-side circuit, at least a part of an artery-side main tube constituting said artery-side circuit and a part of a vein-side main tube constituting said vein-side circuit are formed by the medical tube according to [1] to [10], [22], or [23].
[30] A medical appliance composed of a blood circuit comprising an artery-side circuit and a vein-side circuit,
wherein at least a part of an artery-side main tube constituting said artery-side circuit and a part of a vein-side main tube constituting said vein-side circuit are formed of the medical tube according to [1] to [10], [22], or [23], and said medical tubes are connected to each other via a connecting pipe by at least one means selected from the group consisting of (A) machining, (B) adhesive, and(C) insert molding, or a combination of these means.

### Brief Description of Drawings

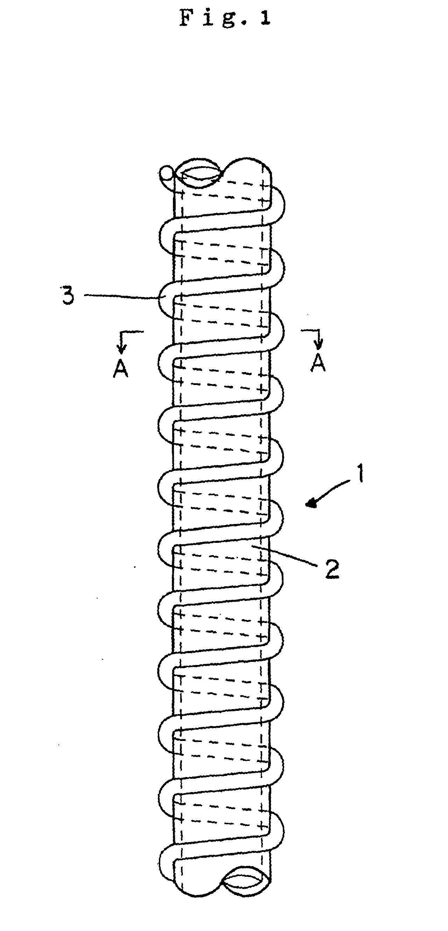

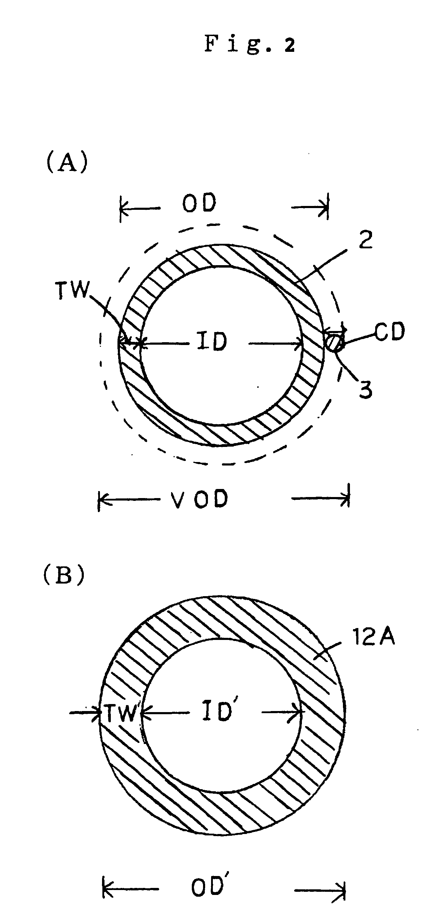

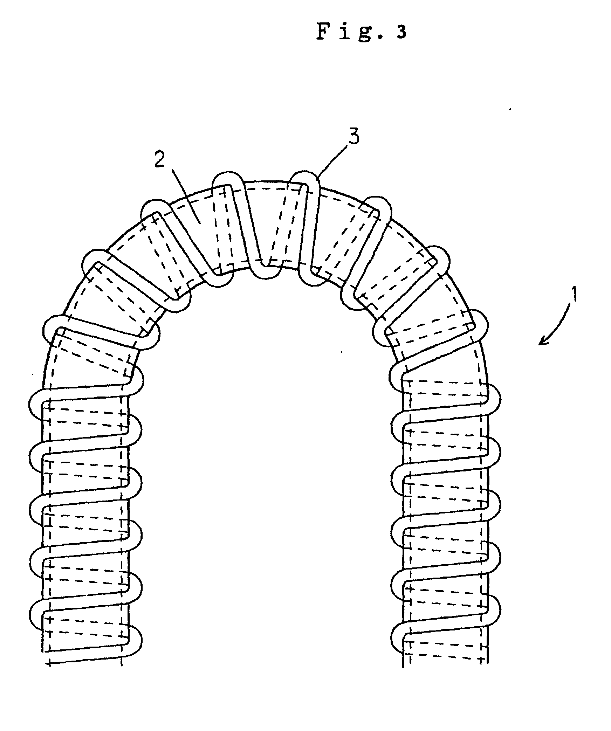

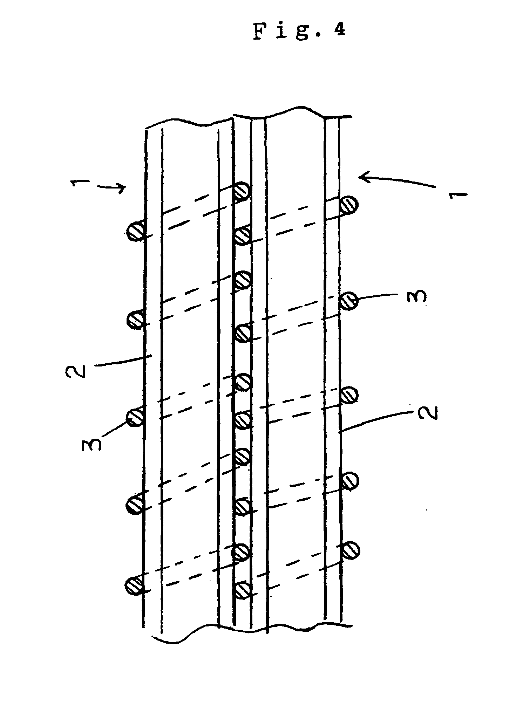

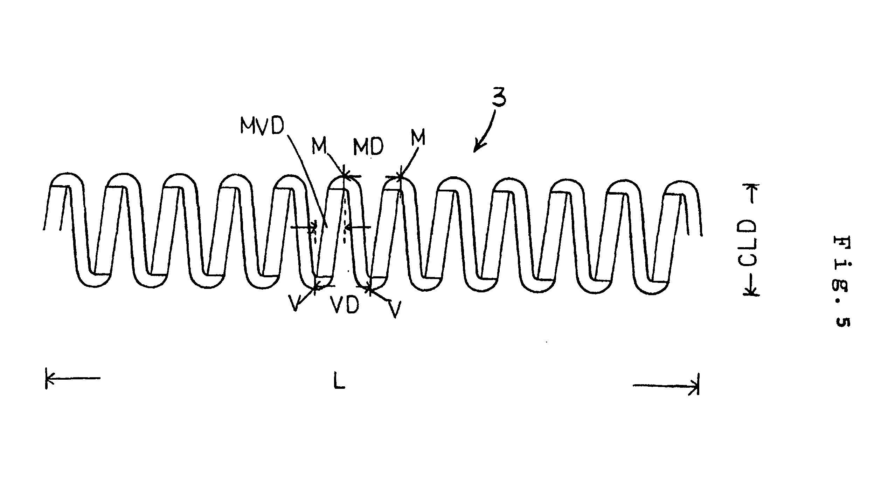

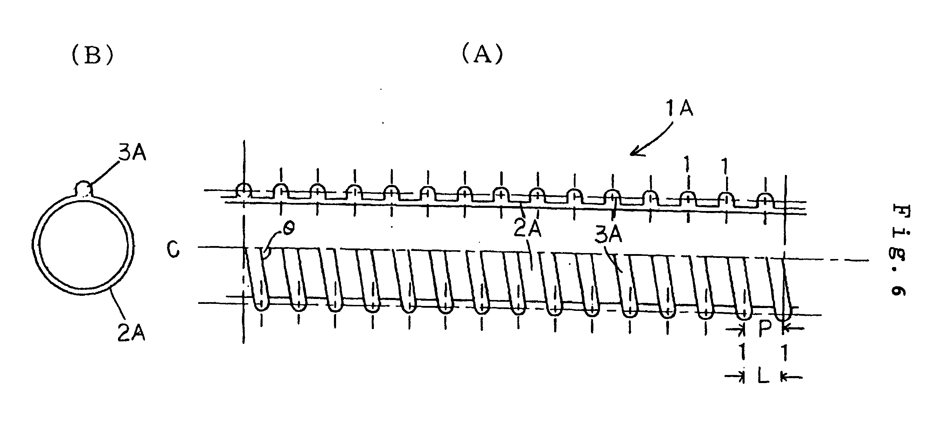

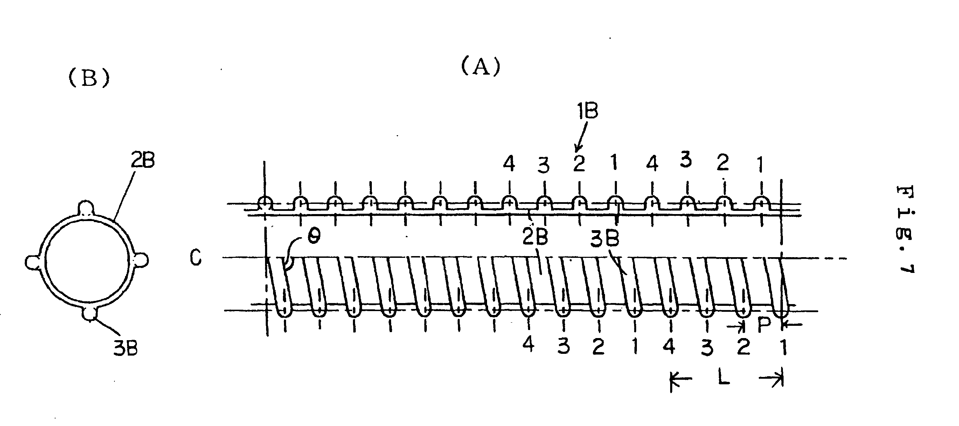

Fig. 1 is a schematic explanatory view of a medical tube of the present invention, Fig. 2(A) is a cross sectional view taken along the A-A line in Fig. 1, Fig. 2(B) is a cross sectional view of a conventional tube as a comparative example, and Fig. 3 and Fig. 4 are explanatory views showing the medical tube shown in Fig. 1 in use. Fig. 5 is a schematic explanatory view showing a coil-shaped reinforcing member, and Fig. 6 is a schematic explanatory view showing a preferred example of the medical tube of the present invention, Fig. 6(A) being a cut-out horizontal cross sectional view of a part thereof and Fig. 6(B) being a vertical cross sectional view thereof. Fig. 7 is a schematic explanatory view showing a preferred example of the medical tube of the present invention, Fig. 7(A) being a cut-out horizontal cross sectional view of a part thereof and Fig. 7(B) being a vertical cross sectional view thereof.
Fig. 8 is a schematic explanatory view of a production device for the medical tube of the present invention. Fig. 9 is a schematic explanatory view showing another example of a production device for the medical tube of the present invention, and Fig. 10 is a schematic explanatory view showing another example of a production device for the medical tube of the present invention.
Fig. 11 and Fig. 12 are schematic explanatory views showing examples of a medical tube on which a bellows-shaped reinforcing member having a coil-shaped concave-convex (or indentation) is mounted, Fig. 11(A) being a cut-out horizontal cross sectional view of a part thereof, Fig. 11(B) being a vertical cross sectional view thereof, Fig. 12(A) being a cut-out horizontal cross sectional view of a part thereof, and Fig. 12(B) being a vertical cross sectional view thereof.
Fig. 13 shows the medical tube of the present invention on which the bellows-shaped reinforcing member is mounted, Fig. 13(A) being a cross sectional view taken along the A-A line in Fig. 11 and Fig. 13(B) being a cross sectional view of a conventional tube 32A, and Fig. 14 and Fig. 15 are views showing this medical tube of the present invention in use.
Fig. 16 is a schematic explanatory view showing an example of the production device for the medical tube of the present invention, Fig. 17 is an enlarged view of a part thereof, and Fig. 18 is an enlarged view of a part of Fig. 17.
Fig. 19 is an explanatory view showing a medical appliance (a blood circuit) in which the medical tube of the present invention is used as constituting members thereof.
Fig. 20 is a schematic explanatory view showing another example of the medical tube of the present invention,
Fig. 21 is a schematic explanatory view showing an example of a production method for the medical tube shown in Fig. 20,
Fig. 22 is a schematic explanatory view showing another example of the medical tube of the present invention, Fig. 23 is a schematic explanatory view showing an example of a production method for the medical tube shown in Fig. 22, and
Fig. 24 is a schematic explanatory view showing an example of the state in which the medical tube of the present invention is connected to a connecting pipe.
Fig. 25 is a schematic explanatory view showing examples of how the medical tube of the present invention with the reinforcing member mounted thereon and a connecting tube are connected to each other, Fig. 26 is a schematic explanatory view showing examples of tip processing of the medical tube of the present invention, and Fig. 27 and Fig. 28 are schematic explanatory views showing examples of how the medical tube of the present invention is connected to a connecting pipe C.

In the drawings, 1, 1A, 1B, 31, 31A denote medical tubes; 2, 2A, 2B, 32, 32A tubes; 3, 3A, 3B, 33, 33A reinforcing members; 101 a medical appliance (a blood circuit); 102 an artery-side circuit; 103 an artery-side main tube; 104 a rolling tube; 105, 115 drip chambers; 116 a washing liquid infusion tube; 107, 117 pressure monitor tubes; 108 a negative pressure detector; 109 a heparin infusion tube; 110, 120 blood processing connectors; 111, 121 shunt connectors; 112 a vein-side circuit; 113 a vein-side main tube; T a connecting pipe; CN a connector; CA a cap; CL a clamp; 50 a production device for the medical tube (tube with the reinforcing member); 52 an extruding device for the tube; 53 an extruding device for the reinforcing member; 55 a molding device for the reinforcing member; 56 a molding die for the reinforcing member; 57 a molding groove for the reinforcing member; 58 a ventilation hole; 59 a ventilation hole; 60 a jig; 61 a groove; 62 a ventilation hole; 63 a vacuum pump; 64 an exhaust pipe; 65 a drive device (for the molding die for the reinforcing member); 66, 67 shafts; 68, 69, 70 gears; 71 a water tank; 72 a take-up machine; 73 a heater; 74 a mouth part die; 220, 250, 270 production devices for the medical tube 1; 221, 251, 271 extruding devices for the tube 2; 222, 252, 272 outer dies; 223, 253, 273 inner dies; 224, 254, 274, 274A, 281A resin material passages; 225, 255, 275 resin material inlets; 226, 256, 276, 286 nozzles; 231, 261 supply devices for the reinforcing member 3; 232, 262, 282 take-up devices for the reinforcing member 3; 233, 263 fixing devices for the take-up device 232; 234, 264, 284 bearings; 264A a supporting jig; 235, 265, 285 a belt; M a motor; 281 an extruding device for the reinforcing member 3; 301 a connecting tube (large-diameter); 302 a connecting tube (small-diameter); 303 a cored bar; 304 a high-frequency welding die; 305, 315 connecting pipes; 311 a molding die; 312 an inner die; 313 an outer die; and 316 a thread groove.

### Preferred Embodiments of the Invention

Hereinafter, the present invention will be explained in detail with reference to the drawings.

Fig. 1 is an explanatory view showing the general idea of a medical tube 1 of the present invention, Fig. 2(A) is a cross sectional view taken along the A-A line in Fig. 1, Fig. 2(B) is a cross sectional view of a conventional tube 12A as a comparative example, and Fig. 3 and Fig. 4 are views showing the medical tube in Fig. 1 in use.

As shown in Fig. 1, a medical tube 1 of the present invention is constituted by mounting a coil-shaped reinforcing member 3 on the outer periphery of a tube 2.

The inner diameter ID of this tube 2, as shown in Fig. 2(A), can be maintained substantially equal in size (for example, about 3.2 to about 4.7 mm) to the inner diameter ID' of the conventional, standard-type tube 12A, which is shown in Fig. 2(B), of a blood circuit line for the same intended use, while the thickness TW can be set and formed to be far smaller compared with the thickness TW' of the conventional tube 12A, for example, 1/2 to 1/3 thereof.

More specifically, the thickness TW of the tube 2 of the present invention can be formed to be far smaller than the thickness TW' (for example, about 1.0 to about 1.3 mm) of the conventional medical tube 12A as a standard-type main tube of the blood circuit line for the same intended use, for example, it can be formed to be about 0.3 mm.

Even when the reinforcing member 3 is formed to have a diameter CD equal to the thickness TW (about 0.3 mm) of the tube 2, the valid outer diameter VOD, including the reinforcing member 3, of the medical tube of the present invention is smaller than the outer diameter OD' of the conventional tube 12A as shown in Fig. 2, and the density of the reinforcing member 3 wound around the tube 2 per unit volume is also small. Consequently, the amount in use of a constituting material of the tube per unit area in a tube cross section and per unit volume in a three-dimensional space of the medical tube 1 can be greatly saved, and in addition, the total weight of the main tube constituting the blood circuit line can be greatly reduced (for example, to a half or less) compared with the conventional tube 12A.

The reduction in thickness TW of the tube 2 does not cause any problem to the medical tube 1 of the present invention in terms of strength since the outer periphery of this tube 2 is protected and reinforced by the reinforcing member 3 mounted in a coil form on the outer periphery of this tube.

Specifically, even when the pressure inside the tube 2 given by fluid (gas, liquid) is locally increased, the increased pressure can be dispersed and absorbed inside the tube. Since the radial expansion of the tube can be suppressed by the reinforcing member 3 mounted on the outer periphery in the coil form. Further, the tube is able to resist the negative pressure inside the tube similarly.

Further, the medical tube of the present invention is characterized by remarkably improved anti-kinking property since this tube has the coil-shaped reinforcing member 3 mounted on the outer periphery thereof. Namely, even when the thickness TW of the tube is small, this tube 2 is not liable to undergo kinking since it smoothly follows the coil-shaped reinforcing member 3 to bend in a curve-shape together with the reinforcing member 3 as shown in Fig. 3, unless it is bent sharply in an extremely narrow range.

Moreover, the medical tube of the present invention has an advantageous effect that high-pressure steam sterilization, even in the state in which the tubes 1 are overlapped with each other as shown in Fig. 4, does not easily cause mutual blocking. Since the outer surfaces of these tubes, which are separated from each other owing to the existence of the coil-shaped reinforcing member 3 mounted on these outer surfaces, are not brought into complete contact or conglutination with each other and the contacting area between the coil-shaped reinforcing members 3 is extremely small as shown in the drawing.

In the medical tube of the present invention, any resin is usable as a material for the tube 2 as long as it is nonrigid plastic (also called flexible synthetic resin) having appropriate flexibility for use as a medical tube. For example, it may be polyolefin or the like such as flexible polyvinyl chloride, polyethylene, and polypropylene which are currently in use, or a material having substantially the same flexibility as they have.

In the present invention, a material used for the coil-shaped reinforcing member 3 may be any plastic as long as it is capable of protecting the tube 2, and though it is preferably rigid or semi-rigid plastic with hardness higher than that of the nonrigid plastic constituting the tube 2, nonrigid plastic or the like having substantially the same flexibility as that of the nonrigid plastic constituting the aforesaid tube 2 is also usable. Here, the reinforcing member 3, even though being hard itself, when it is mounted on the tube 2, only has to have such hardness to be capable of bending the entire medical tube 1 on which it is formed.

The combination of the materials used for the tube 2 and the coil-shaped reinforcing member 3 can be determined by appropriate selection in consideration of the correlations among the respective hardness of the tube 2 and the coil-shaped reinforcing member 3, the thickness TW, and the diameter CD, mutual rebound resilience between the tube 2 and the reinforcing member 3, the hardness of the whole medical tube 1 (elasticity), and so on.

In the present invention, the form of the reinforcing member is not specifically limited as long as it can give a reinforcing function to the tube on which it is mounted, a mono-filament or a small-diameter tubular body can be named as an example. Here, the "mono-filament" or the "small-diameter tubular body" includes a reinforcing member whose cross section has a circular shape (circle, ellipse, semi-circle), an angular shape (triangle, square, pentagon, hexagon), a trapezoidal shape, and so on. A perfect circle or a circular shape approximate to a perfect circle is preferable. Further, the shape of the reinforcing member 3 is freely changeable according to its intended use. More specifically, for example, the pitch of the coil, the winding form thereof, the winding density thereof (namely, the distance MD between neighboring mountains M, the distance VD between neighboring valleys V, the distance MVD between the mountain M and the valley V, and the number of times the coil is wound per unit length L, as shown in Fig. 5), the diameter CLD of the coil, and so on are freely changeable in design according to the intended use. Thus, the "coil" in the present invention includes all the forms in which it is wound around the outer periphery of the tube 2. Incidentally, the diameter CD of the reinforcing member 3 as shown in Fig. 2 can be determined in consideration of the hardness (elasticity) of the entire medical tube, and it may be larger or smaller than the thickness TW of the tube 2 or substantially the same.

Fig. 6 and Fig. 7((A) each is a cut-out horizontal cross sectional view of a part and (B) each is a vertical cross sectional view) are schematic explanatory views showing preferred examples of the medical tube of the present invention. A medical tube 1A shown in Fig. 6 is so constructed that one continuous coil-shaped reinforcing member 3A is wound around the outer periphery of the tube 2A with regular pitches P. A medical tube 1B shown in Fig. 7 is so constructed that a plurality of (for example, 4 in Fig. 7) continuous coil-shaped reinforcing members 3B are wound around the outer periphery of the tube 2B in sequence with regular pitches P respectively.

In the explanation to follow, the medical tube 1, the tube 2, and the reinforcing member 3 include all the forms of the medical tube 1A and the medical tube 1B, the tube 2A and the tube 2B, and the reinforcing member 3A and the reinforcing member 3B, in Fig. 6 and Fig. 7 respectively, and therefore, A and B will be omitted.

As a preferred example of the present invention, the thickness TW of the tube 2 is set to 0.2 to 0.7 mm, preferably 0.3 to 0.5 mm.

Further, the diameter CD of the reinforcing member 3 is set to 0.3 to 1.5 mm, preferably 0.7 mm.

In short, in the example of the present invention, it is preferable that the sum of the thickness TW of the tube 2 and the diameter CD of the reinforcing member 3 is substantially equal to or smaller than the thickness TW' (generally about 1.0 to about 1.3 mm) of the conventional tube 12A.

The pitch P (namely, substantially the same as MD and VD in Fig. 5) of the coil of the reinforcing member 3 in the present invention is set to 2.0 to 5.0 mm, preferably 3.0 mm.

The number of the coils NC of the coil-shaped reinforcing member 3 wound around the outer periphery of the tube 2 is 1 to 10, preferably 2 to 8, more preferably 4 to 6. The larger the number of the coils NC is, the more preferable, since higher efficiency in winding the reinforcing member around the outer periphery of the tube can be obtained in a later described production process in which the reinforcing member 3 is mounted in the coil form on the outer periphery of the tube 2. But when the number of the coils is too large, exceeding, for example, 10, it is not preferable since the density of the reinforcing member on the outer periphery of the tube becomes too high to increase the total weight. Further, when the number of the coils NC is too small, for example, one, it is not very preferable since the speed of winding the reinforcing member around the outer periphery of the tube becomes low to lower production efficiency similarly in the mounting process. In any of those cases, however, the object of the present invention can be achieved when the conditions such as the aforesaid pitch P and a lead L are satisfied.

The lead L of the reinforcing member 3 shown in Fig. 6 and Fig. 7 is set to 12.0 mm when, for example, the number of the coils NC is 4 and the pitch P is 3.0 mm, and is set to approximately 3.0 mm when the number of the coils NC is one and the pitch P is 3.0 mm.

Accordingly, the coil-shaped reinforcing member 3 of the tube 2 is so formed that the pitch P is within the range of 2.0 to 50.0 mm, considering the aforesaid range of the pitch P and the number of the coils NC.

Further, in the present invention, it is suitable that a winding angle θ of the coil-shaped reinforcing member 3 relative to the center line (axial line) C in the longitudinal direction of the tube 2 is set to 60 degrees to 85 degrees, preferably 75 degrees to 85 degrees, as shown in Fig. 6 and Fig. 7.

The winding angle θ less than 60 degrees is not preferable since such winding angle θ is too small to lower anti-kinking property. Specifically, when the winding angle θ is too small, it causes the cross section of the tube 2 to have the shape of an ellipse or a flatter shape than an ellipse when the medical tube 1 is bent, so that elasticity is lost and anti-kinking property is lowered. It is preferable that the winding angle θ is as close to 90 degrees as possible within the aforesaid range. When the angle is close to 90 degrees, the cross section of the tube 2 can be maintained in a perfect circle or the shape approximate to a perfect circle when the tube 1 is bent so that the tube 2 has elasticity and its anti-kinking property is improved. Incidentally, the winding angle θ exceeding 85 degrees is not preferable in the production of the medical tube 1 since this disenables the coil-shaped reinforcing member 3 to be wound around the outer periphery of the tube 2.

Even in the case of the winding angle θ smaller than the aforesaid range, anti-kinking property can be improved when the thickness TW of the tube 2 and the diameter CD of the coil-shaped reinforcing member 3 are made large. But this leads to the increase in the total weight of the medical tube 1, and therefore, does not suit the object of the present invention. Note that the winding angle θ can be freely set depending on the speed of extruding (supplying) the coil-shaped reinforcing member 3 in the production process thereof as will be described later. The decrease in the extruding speed increases the winding angle θ, and conversely, the increase in the extruding speed reduces the winding angle θ.

In the medical tube of the present invention, it is suitable that the coil-shaped reinforcing member 3 covering the surface of the tube 2 is formed to occupy an area S of at least 30% or larger, preferably 40% or larger, more preferably 50% or larger, or still more preferably 60% to 70% or larger.

The occupying area S far smaller than the above is not preferable since it causes the density of the reinforcing member 3 covering the surface of the tube 2 to be too low so that anti-kinking property is lowered, while the occupying area S which is too large is not preferable since it causes the reinforcing member 3 to cover the outer periphery of the tube 2 so densely that the total weight becomes too heavy.

As described above, in the medical tube of the present invention, by setting the pitch P, the lead L, the occupying area S, and the winding angle θ of the coil-shaped reinforcing member 3 covering the surface of the tube 2, it can cover the surface of the tube 2 in a spring-shape with a certain degree of density while maintaining an appropriate degree of elasticity.

Further, in the present invention, the thickness of the tube 2 can be reduced depending on the correlations among the respective hardness of the tube 2 and the coil-shaped reinforcing member 3, the thickness TW, and the diameter CD, and thereby the medical tube suitable for a blood circuit line having an appropriate elasticity, anti-kinking property, and the like can be designed and produced. Preferred examples of the correlations among the respective hardness of the tube 2 and the coil-shaped reinforcing member 3, the thickness TW, the diameter CD, and so on will be listed below.

When the conditions are assigned as follows:
a. three conditions of thin (0.2 mm to less than 0.4 mm), intermediate (0.4 mm to less than 0.6 mm), and thick (0.6 mm to 0.7 mm) are assigned for the thickness TW of the tube 2;
b. three conditions of hard, intermediate, and soft are assigned for the hardness TH of this tube 2; and
c. meanwhile, three conditions of small (0.3 mm to less than 0.7 mm), intermediate (0.7 to less than 1.2 mm), and large (1.2 mm to 1.5 mm) are assigned for the diameter CD of the reinforcing member 3; and
d. three conditions of hard, intermediate, and soft are assigned for the hardness CH of the reinforcing member 3, condition settings, for example, as follows are possible:
   (1) the thickness TW is thin, the hardness TH is hard, the diameter CD is large, and the hardness CH is soft;
   (2) the thickness TW is intermediate, the hardness TH is intermediate, the diameter CD is intermediate, and the hardness CH is intermediate; and
   (3) the thickness TW is thick, the hardness TH is soft, the diameter CD is small, and the hardness CH is hard.

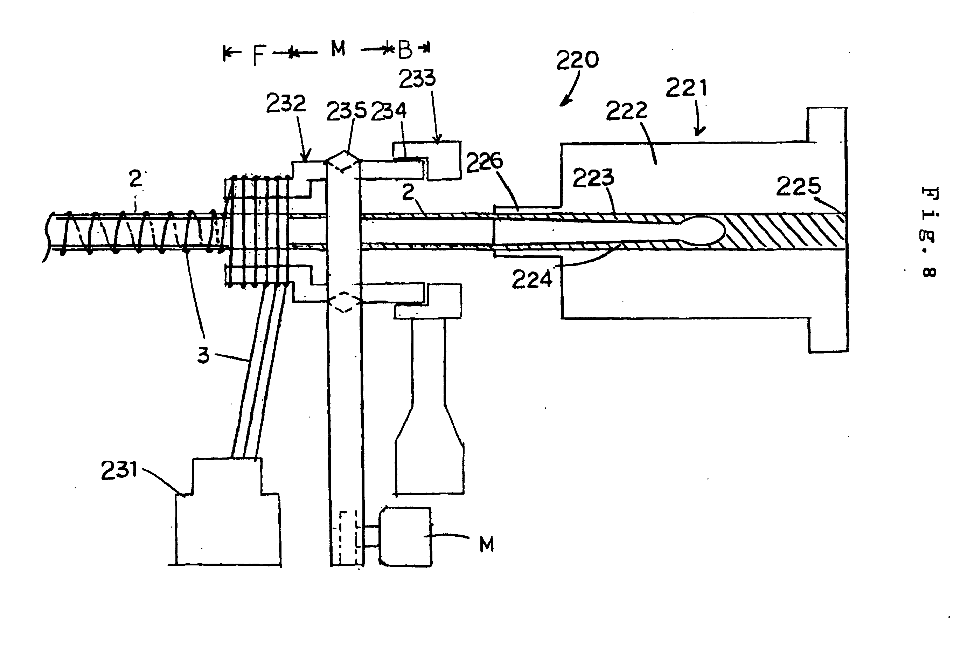

Fig. 8 to Fig. 10 are schematic explanatory views showing examples of a production device for the medical tube of the present invention. In Fig. 8, which is a schematic explanatory view showing an example of a production device 220 for the medical tube of the present invention, the production device 220 includes an extrusion die 221 (222 denotes an outer die and 223 denotes an inner die) for the tube 2, a take-up device 232 for the reinforcing member 3, and a fixing table 233 for the take-up device 232. A backward portion B of the take-up device 232 is rotatably mounted on the fixing device 233 via a bearing 234, and a belt 235 is mounted on a middle portion M of the take-up device 232 so as to transmit the rotation of a motor M thereto to enable the take-up device 232 to rotate. Further, the reinforcing member 3 is wound around the outer periphery of a forward portion F of the take-up device 232.

The medical tube 1 of the present invention can be produced by this device, for example, in the following manner.

The hot-melted material of the tube 2 is injected from a resin material inlet 225 to be extruded from a nozzle 226 in a tube form via a resin material passage 224. Meanwhile the reinforcing member 3 is fed from a supply device 231 for the reinforcing member 3 to be taken up around the outer periphery of the forward portion F of the take-up device 232 to which the rotation is applied by the motor M, and is sent in the extruding direction of the tube 2 in sequence to be mounted on the outer periphery of the tube 2.

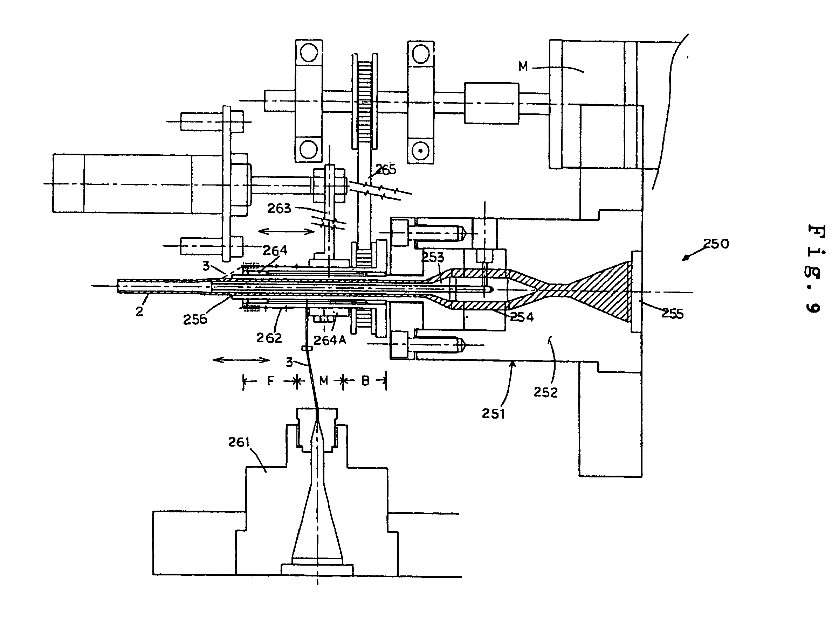

Fig. 9 is a schematic explanatory view showing another example of the production device of the medical tube 1. In Fig. 9, the structure of a production device 250 and a production method thereof are substantially the same compared with the production device 220 in Fig. 8 except that: (a) the outer periphery in the vicinity of a middle portion M of a take-up device 262 is rotatably mounted on a fixing device 263 via a supporting jig 264A; (b) an inner die 253 extends to an inner side of the take-up device 262, and a bearing 264 is interposed between the take-up device 262 and the inner die 253 so that the take-up device 262, while rotating, takes up the reinforcing member 3 in the direction from the vicinity of the middle portion M to a forward portion F, thereby mounting the reinforcing member 3 on the outer periphery of the tube 2 extruded from a nozzle 256; (c) the reinforcing member 3 is extruded and supplied from an extrusion die 261 for the reinforcing member; and so on, and therefore, detailed explanation thereof will be omitted.

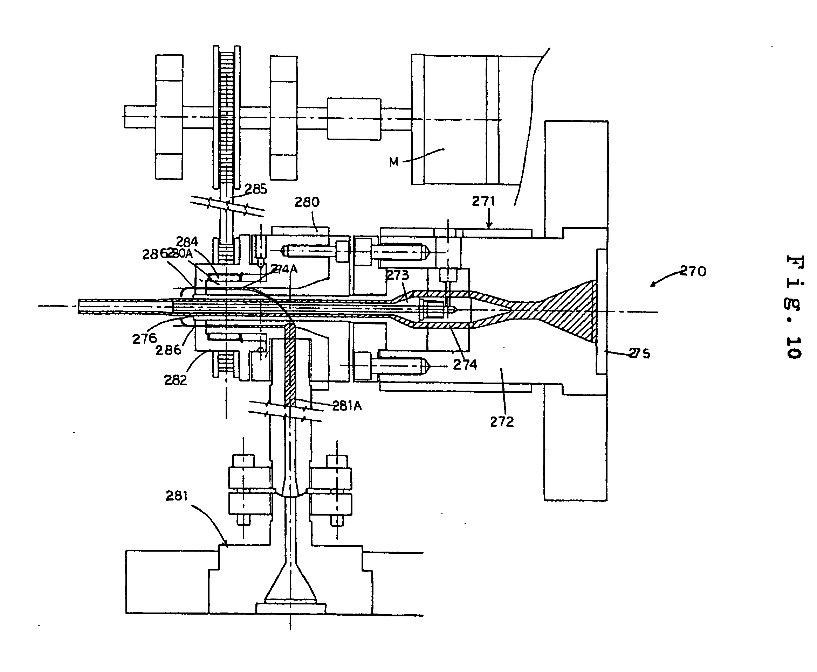

Further, Fig. 10 is a schematic explanatory view showing another example of the production device for the medical tube 1 of the present invention. A production device 270 in Fig. 10 is different compared with the production devices 220, 250 in Fig. 8 and Fig. 9 only in that: (a) an extrusion die 281 for the reinforcing member 3 is provided instead of the supply devices 231, 261 for the reinforcing member 3; (b) a passage 274A for the constituting material of the reinforcing member 3 which is supplied from the extrusion die 281 for the reinforcing member is provided in a forward direction of the extrusion die 271, and a mouth part die 280 with the aforesaid inner die 273 extended therein is provided; (c) further, a take-up device 282 is mounted on a forward portion of this mouth part die 280, and a bearing 284 is interposed between the inner side of the take-up device 282 and a forward portion 280A of this mouth part die 280; (d) further, the tube 2 is extruded from a nozzle 276 of the inner die 273, and the constituting material of the reinforcing member 3 which is supplied from the extrusion die 281 is extruded from a nozzle 286 (note that the number thereof is adjustable depending on the number of the coils NC of the reinforcing member 3) while the take-up device 282 is rotated, thereby winding the constituting material of the reinforcing member 3 around the outer periphery of the tube 2; and so on, the other structure being substantially the same, and therefore, the detailed explanation thereof will be omitted.

An example of a method for producing the medical tube 1 by the production device 270 is, for example, as follows.

The hot-melted material of the tube 2 is injected from a resin material inlet 275 to be extruded from the nozzle 276 via a resin material passage 274.

Meanwhile, the constituting material of the reinforcing member 3 is extruded from the extrusion die 281 and passes a passage 281A and a passage 274A of the mouth part die 280 to be extruded from the nozzle 286 of the take-up device 282 to which rotation is applied by a motor M, and at the same time it is sent in the extruding direction of the tube 2 in sequence, to be mounted on the outer periphery of the tube 2.

The reinforcing member 3 can be easily fixed onto the outer periphery of the tube 2 since the tube 2 has been just extruded and is in a hot state at the time of the mounting. Incidentally, the reinforcing member 3 is preferably heated by a heater or the like in order to fix the reinforcing member 3 onto the tube 2 with higher reliability.

Further, in order to realize still higher reliability in fixing the reinforcing member 3 onto the tube 2, a temperature adjusting device for heating the tube 2 and the reinforcing member 3 can be disposed in a position where the fixing of the tube 2 and the reinforcing member 3 is carried out.

The shape of the coil-shaped reinforcing member 3 fixed onto the outer periphery of the tube 2 can be freely set by appropriately adjusting the winding density of the reinforcing member 3 on the take-up devices 232, 262, 282, the rotation speed of the take-up devices 232, 262, 282, the speed at which the tube 2 is extruded, and so on.

In the medical tube of the present invention, it is also a preferable embodiment in which the reinforcing member is a bellows-shaped reinforcing member having a coil-shaped concave-convex(or indentation).

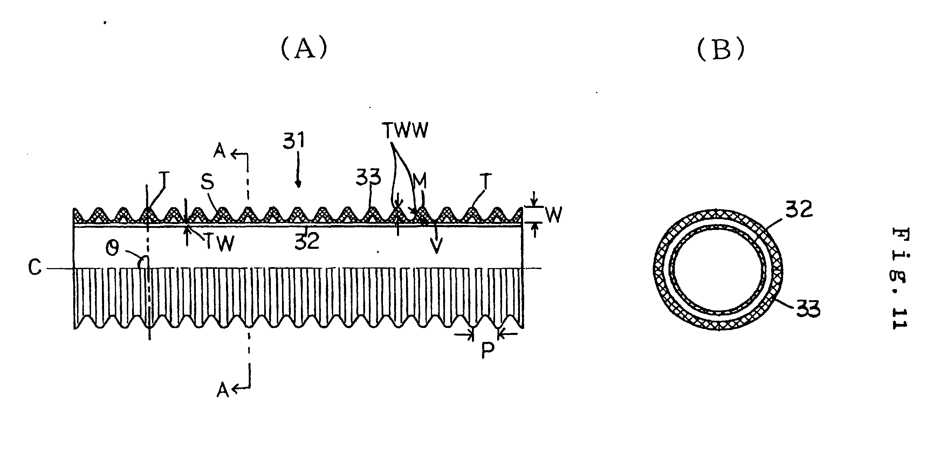

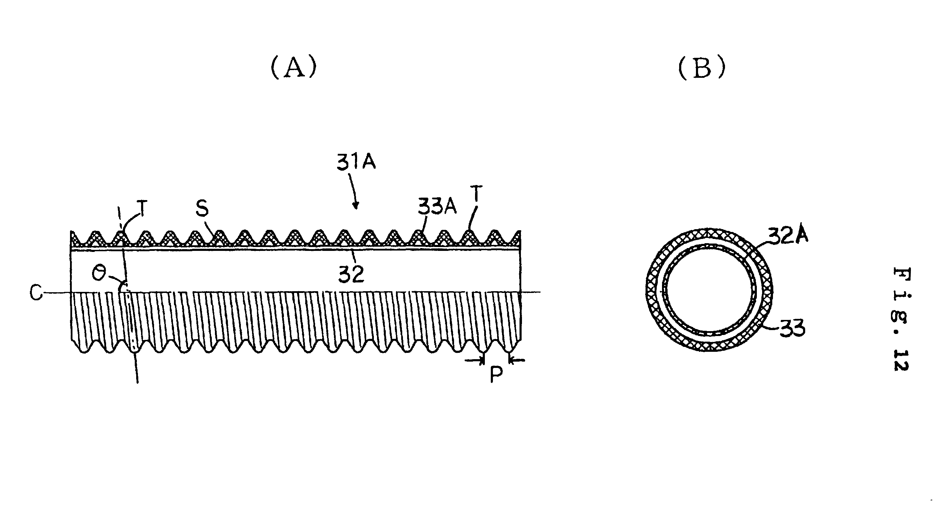

Fig. 11 and Fig. 12 are schematic explanatory views showing examples of a medical tube on which such a bellows-shaped reinforcing member having a coil-shaped indentation is mounted, (A) each being a cut-out horizontal cross sectional view of a part thereof and (B) each being a vertical cross sectional view thereof, respectively.

In Fig. 11, a medical tube 31 with a reinforcing member mounted thereon is so constructed that, on the outer periphery of a tube 32, a tube-shaped reinforcing member 33 is molded to have a continuous indentation by vacuum absorption in a later described production process, and is formed to be the bellows-shaped reinforcing member in which this indentation has a predetermined pitch P and an angle θ.

In the present invention, the pitch P of the indentation is a distance between a protruding portion T and a protruding portion T as shown in (A) in the drawings. Further, the angle θ is the angle θ made by a center line (an axial line) C in the longitudinal direction of the tube 32 and the aforesaid protruding portion T.

In Fig. 11, the medical tube 31 is so constructed that the angle θ is 90 degrees, and in Fig. 12, the medical tube 31A is so constructed that the angle θ is smaller than 90 degrees.

Incidentally, in Fig. 11 and Fig. 12, the medical tubes 31, 31A are so constructed that a small space S is left between the tube 32 and each of the bellows-shaped reinforcing members 33, 33A having the coil-shaped indentation. Meanwhile they may be so constructed that the tube 32 and each of the bellows-shaped reinforcing members 33, 33A having the coil-shaped indentation are in close contact with each other to eliminate the space S between the tube 32 and each of the bellows-shaped reinforcing members 33, 33A having the coil-shaped indentation.

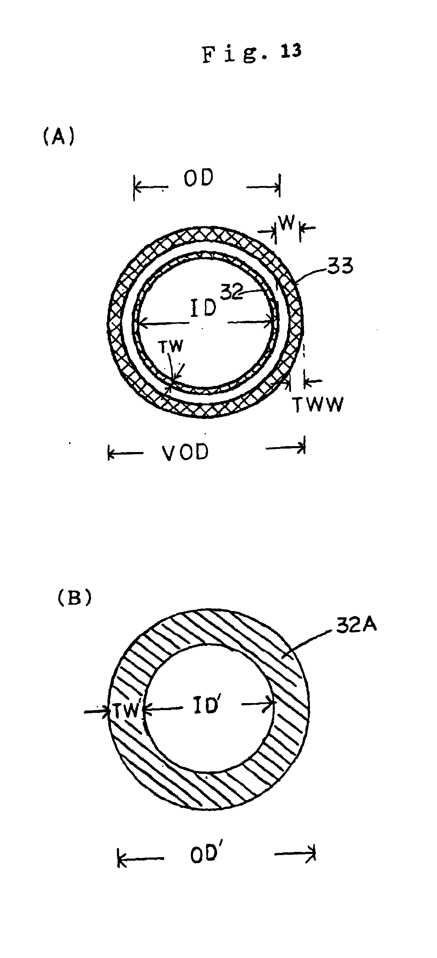

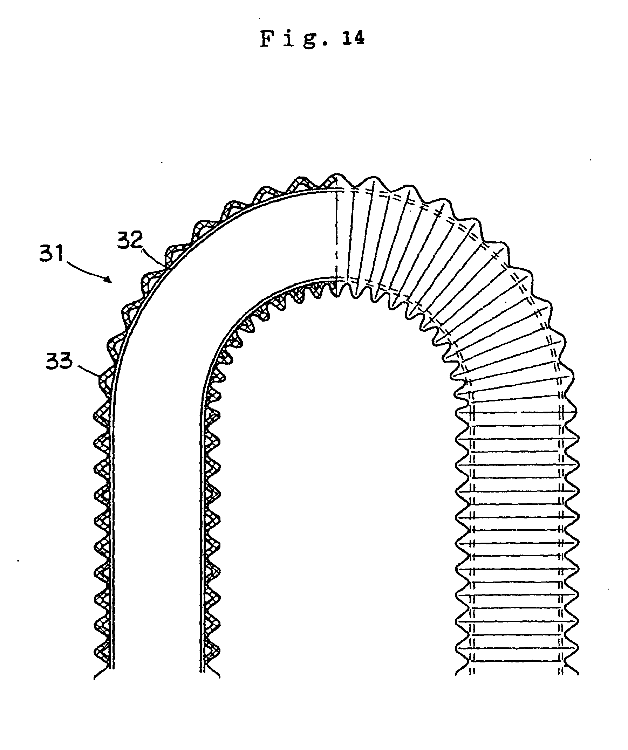

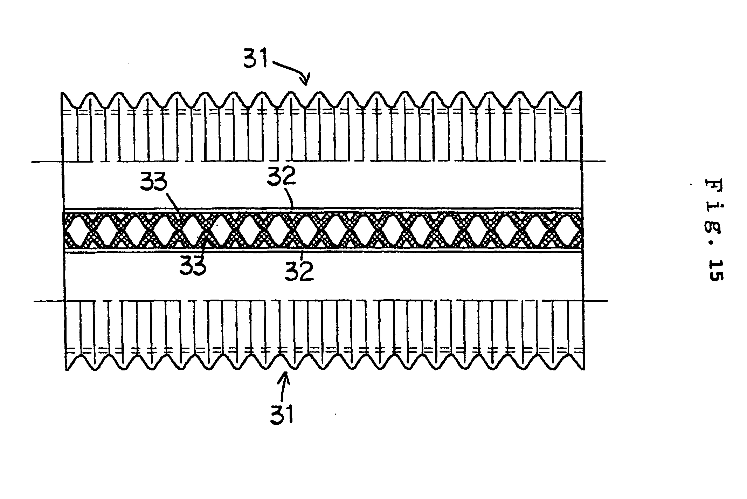

Fig. 13 shows the medical tube of the present invention with the bellows-shaped reinforcing member mounted thereon, Fig. 13(A) being a cross sectional view taken along the A-A line in Fig. 11 and Fig. 13(B) being a vertical cross sectional view of a conventional tube 32A, and Fig. 14 and Fig. 15 are views showing this medical tube of the present invention in use.

In the medical tube of the present invention in the case where the bellows-shaped reinforcing member having the coil-shaped indentation is mounted thereon, this bellows-shaped reinforcing member is a controlling factor of most of the performances of the tube such as flexibility (bending elasticity) of the entire tube and anti-kinking property, just as the case where the reinforcing member made of the mono-filament or the small-diameter tubular body is mounted thereon. While the inner peripheral surface of the tube 32 is similarly formed to be flat so that fluid such as blood can flow smoothly to cause no deposition of a thrombus or the like. Consequently, the tube 32 is not liable to undergo kinking when it bends following the reinforcing member 33, and the thickness TW can be made small to the limit within the range allowing the tube 32 to resist the inner pressure of the fluid (gas, liquid).

Further, while it is possible to maintain the inner diameter ID of the tube 32 substantially equal in size (3.2 to 4.7 mm) to that of the conventional standard-type tube 32A of a blood circuit line for the same intended use, just as the case of the reinforcing member such as the mono-filament, and it also is possible to make the thickness TW of the tube 32 smaller, for example, 1/2 to 1/3 or less compared with the conventional tube 32A.

For example, the thickness TW of the tube 32 can be made smaller, for example, approximately 0.3 mm, than the thickness TW' (1.0 o 1.3 mm) of the conventional medical tube 32A as a standard-type main tube of the blood circuit line for the same intended use.

More specifically, as shown in Fig. 11(A) and Fig 13(A), even when a width W of the reinforcing member 33 (the distance from a mountain M (the top portion of the protruding portion T) to the valley V (the surface of the reinforcing member 33 in contact with the tube 32) of the indentation) and/or the thickness TWW (the thickness of a cross section in a vertical direction or an oblique direction of the reinforcing member 33) are(is) made equal to the thickness TW (approximately 0.3 mm) of the tube 32, a valid(effective) outer diameter VOD of the medical tube 31 of the present invention including the reinforcing member 33 is smaller than the outer diameter OD' of the conventional tube 32A, and the density of the reinforcing member 33 wound around the tube 32 per unit volume is also small. Consequently, the amount of the constituting material of the tube in use per unit area in a tube cross section and per unit volume in a three-dimensional space of the tube 31 with the reinforcing member mounted thereon can be greatly saved (reduced), and in addition, the total weight of the main tube comprising the blood circuit line can be greatly reduced compared with the conventional tube 32A (to a half or less), just like the case when the reinforcing member such as that in the mono-filament shape is mounted.

Further, similarly to the case of the reinforcing member in the mono-filament shape and so on, the medical tube 31 with the bellows-shaped reinforcing member does not have any problem in terms of strength even when the thickness TW of the tube 32 is made small since the outer periphery of the tube 32 is protected by the reinforcing member 33.

In other words, even when the pressure given by the fluid (gas, liquid) is locally increased inside the tube 32, the reinforcing member 33 can suppress the radial expansion of the tube 32 so that the pressure can be dispersed and absorbed inside the tube 32 and the tube also is able to resist the negative pressure inside the tube 32 similarly.

Further, just as the case of the reinforcing member in the mono-filament shape, anti-kinking property is remarkably improved in this medical tube since the bellows-shaped reinforcing member 33 having the coil-shaped indentation is mounted on the outer periphery of the tube 32. More specifically, like the case shown in Fig. 3, even when the thickness TW of the tube 32 is small, it is not liable to undergo kinking, since the tube 32 bends in a curve form following the bellows-shaped reinforcing member 33 having the coil-shaped indentation as shown in Fig. 14 unless it is bent sharply in an extremely small range.

Further, in the medical tube with the bellows-shaped reinforcing member mounted thereon, high-pressure steam sterilization even in the state in which the tubes 31 are overlapped with each other does not easily cause mutual blocking since the contacting area of the coil-shaped reinforcing members 33 is extremely small as shown in Fig. 15, similarly to the previous description on Fig. 4 in the case of the reinforcing member in the mono-filament shape.

The aforesaid flexible (nonrigid) plastic is also used as the material used for the tube 32. The inner diameter ID of the tube 32 can be set to substantially the equal value to that of the conventional tube for the same intended use, while the thickness TW thereof can be set smaller than that of the conventional tube, for example, to 1/2 to 1/3 or less.

As the material used for the bellows-shaped reinforcing member 33 having the coil-shaped indentation, are nonrigid plastic, semi-rigid plastic, rigid plastic, or the like which can be extruded at the same time with the aforesaid nonrigid plastic constituting the tube 32 in a later described production process and which has substantially the same flexibility. Even when semi-rigid or rigid plastic is used, the thickness TWW thereof can be made small to the limit as long as, in a word, it can bend along the coil-shaped indentation and can be reinforced by the coil-shaped indentation so as not to be damaged when it bends. In short, any material of reinforcing member is used as the reinforcing member 33 as long as it can protect the tube 32. The combination of the materials used for the tube 32 and the bellows-shaped reinforcing member 33 having the coil-shaped indentation can be determined by appropriate selection in consideration of the correlations among the respective hardness of the tube 32 and the reinforcing member 33, the thickness TW, the width W, the thickness TWW, mutual rebound elasticity between the tube 32 and the reinforcing member 33, and the hardness (elasticity) of the entire medical tube 31.

In the present invention, "the bellows shape having the coil-shaped indentation" includes all the forms as long as they are in a bellows-shape. More specifically, the shape of the cross section of the bellows-shaped reinforcing member 33 having the coil-shaped indentation is not especially limited, but it is preferably a perfect circle or a circular shape approximate to a perfect circle. Further, the shape (pitch P and angle θ) and the like of the reinforcing member 33 can be freely changed in design according to its intended use. The width W and the thickness TWW of the reinforcing member 33, which can be also determined in consideration of the hardness (elasticity) of the entire tube 31 with the reinforcing member, may be larger or smaller than the thickness TW of the tube 32, or substantially equal thereto.

As a preferred example, the thickness TW of the tube 32 is set to 0.2 to 0.7 mm, preferably 0. 3 to 0.5 mm.

Meanwhile, the width W and/or the thickness TWW of the reinforcing member 33 are(is) set to 0.3 to 1.5 mm, preferably 0.5 mm.

In short, it is only necessary that the sum of the thickness TW of the tube 32, the width W and/or the thickness TWW of the reinforcing member 33 is substantially equal to or smaller than 1.0 to 1.3 mm, which is the thickness TW' of the conventional tube 32A.

The pitch P of the reinforcing member 33 of the present invention is set to 1.0 to 5.0 mm, preferably 1.5 mm.

It is suitable that the bellows-shaped reinforcing member is so formed that the angle θ made by the center line (axial line) C in the longitudinal direction of the tube 32 and the protruding portion T is 60 degrees to 90 degrees, preferably 90 degrees.

The angle θ smaller than 60 degrees is not preferable since the angle θ is so small that anti-kinking property is lowered. Specifically, the small angle θ causes the cross section of the tube 32 to have the shape of an ellipse or a flatter shape than an ellipse when the tube 31 with the reinforcing member mounted thereon is bent, so that elasticity is lost and anti-kinking property is lowered. It is more preferable that the angle θ is 90 degrees or as close to 90 degrees as possible. When the angle is close to 90 degrees, the cross section of the tube 32 can be maintained in the shape of a perfect circle or the shape approximate to a perfect circle when the tube 31 with the reinforcing member mounted thereon is bent so that the tube 31 has elasticity and anti-kinking property is improved.

On the other hand, when the angle θ is small within the aforesaid range, anti-kinking property can be improved by increasing the thickness TW of the tube 32 or increasing the width W and/or the thickness TWW of the bellows-shaped reinforcing member 33 having the coil-shaped indentation.

As described above, in the case of mounting the bellows-shaped reinforcing member, by setting appropriate values of the pitch P and the angle θ of the bellows-shaped reinforcing member 33 having the coil-shaped indentation which covers the surface of the tube 32, the bellows-shaped reinforcing member can completely cover the surface of the tube 32 in a spring-shape while maintaining an appropriate degree of elasticity.

Further, when the correlations among the respective hardness of the tube 32 and the bellows-shaped reinforcing member 33 having the coil-shaped indentation, the thickness TW, the width W, and the thickness TWW are appropriately set, the medical tube 31 of the present invention suitable for the blood circuit line can be produced, in which the thickness TW of the tube 32 is sufficiently small and the entire medical tube 31 has an appropriate degree of elasticity, anti-kinking property, and so on.

Examples of the correlations among the respective hardness of the tube 32 and the bellows-shaped reinforcing member 3 having the coil-shaped indentation, the thickness TW, the width W, and the thickness TWW will be listed below.

When the conditions are assigned as follows:
a. three conditions of thin (0.2 mm to less than 0.4 mm), intermediate (0.4 mm to less than 0.6 mm), and thick (0.6 mm to 0.7 mm) are assigned for the thickness TW of the tube 32;
b. three conditions of hard, intermediate, and soft are assigned for the hardness TH of this tube 32; and
c. three conditions of small (0.3 mm to less than 0.7 mm), intermediate (0.7 mm to less than 1.2 mm), and large (1.2 mm to 1.5 mm) are assigned for the width W and/or the thickness TWW of the reinforcing member 33; and
d. three conditions of hard, intermediate, and soft are assigned for the hardness CH of the reinforcing member 3, condition settings, for example, as follows are possible:
   (1) the thickness TW is thin, the hardness TH is hard, the width W and/or the thickness TWW are(is) large, and the hardness CH is soft;
   (2) the thickness TW is intermediate, the hardness TH is intermediate, the width W and/or the thickness TWW is(are) intermediate, and the hardness CH is intermediate; and
   (3) the thickness TW is thick, the hardness TH is soft, the width W and/or the thickness TWW are(is) small, and the hardness CH is hard.

The medical tube with the bellows-shaped reinforcing member mounted thereon can be produced, for example, by the following production device.

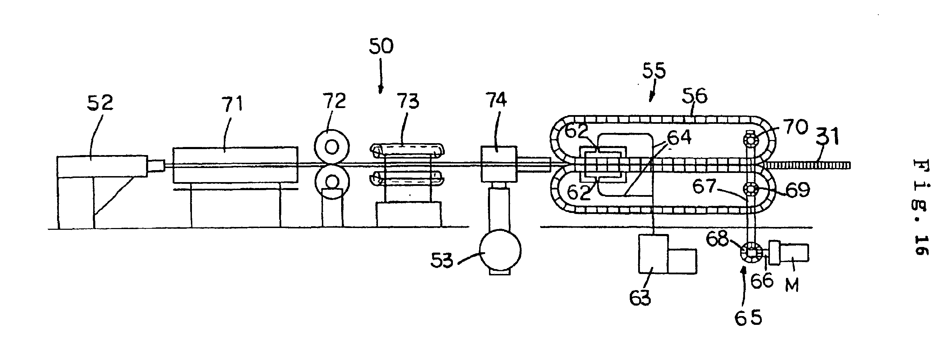

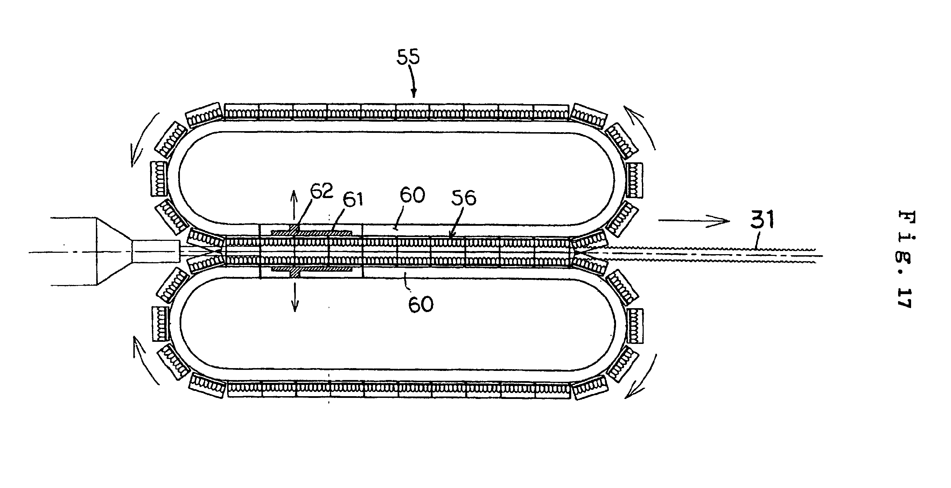

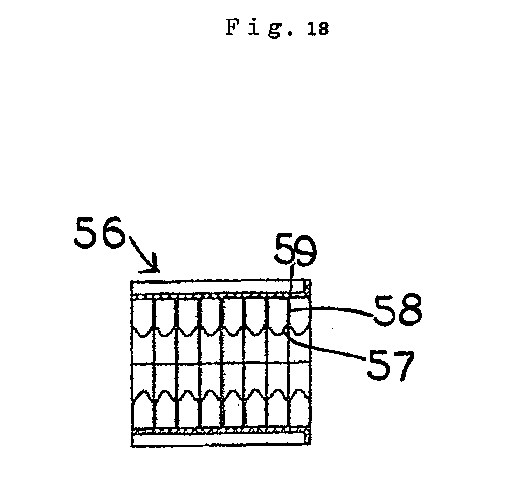

Fig. 16 is a schematic explanatory view showing an example of a production device for the medical tube 31 of the present invention, Fig. 17 is an enlarged view of a part thereof, and Fig. 18 is an enlarged view of a part of Fig. 17.

A production device 50 for the medical tube 31 of the present invention is composed of an extruding device 52 for the tube 32, an extruding device 53 for the reinforcing member 33, and a molding device 55 for molding the extruded reinforcing member 33 in the bellows shape.

To describe in more detail, a mouth part die 74 is interposed between the aforesaid extruding device 52 for the tube 32 and molding device 55 for the reinforcing member 33, and the extruding device 53 for the reinforcing member 33 is disposed beside the mouth part die 74. Moreover, when necessary, a water tank 71, a take-up machine 72, and a heater 73 are provided between the extruding device 52 for the tube 32 and the mouth part die 74.

The molding device 55 for the reinforcing member is composed of molding dies 56 for molding the reinforcing member in the bellows-shape, jigs 60 for the molding dies 56, a vacuum pump 63, and a drive device 65 for the molding dies 56.

A pair of the above jigs 60 are disposed to face each other as shown in Fig. 17, and the molding dies 56 are movably disposed on the outer peripheries of these jigs 60. To describe in more detail, the jigs 60 are disposed in two places to face each other, being apart from each other with a space therebetween to which the aforesaid constituting materials of the tube 32 and the reinforcing member 33 are extruded. The molding dies 56 are movably (rotatably) disposed on the outer peripheries of the jigs 60 respectively. A drive device 65 controls the molding dies 56 facing each other as well as the jigs 60 so as to vary the moving (rotating) speed according to the pitch P, the angle θ, and so on of the coil-shaped indentation which is to be shaped in the reinforcing member 33.

The drive device 65 is constructed, for example, as shown in Fig. 16, by mounting a gear 68 on a motor M via a shaft 66, mounting a shaft 67 on this gear 68, and mounting gears 69, 70 on this shaft 67. The rotation of the motor M is transmitted to these gears 69, 70 via the shaft 66, the gear 68, and the shaft 67 so that the gears 69, 70 rotate the aforesaid pair of the molding dies 56 in the direction of extruding the tube 32 and the reinforcing member 33 (note that, in Fig. 17, the upper molding die 56 is rotating anticlockwise and the lower molding die 56 is rotating clockwise).

In the present invention, the drive device 65 is not limited to that shown in Fig. 16, and in a word, any drive device may be used as long as it can rotate the pair of the molding dies 56 in the direction of extruding the tube 32 and the reinforcing member 33, thereby enabling the molding dies 56 to sandwich the tube 32 and the reinforcing member 33 therebetween, and also to form the coil-shaped indentation in the constituting material of the reinforcing member 33 into the bellows-shape.

The molding dies 56 are constructed by integrating a plurality of dies, and as shown in Fig. 18, it has ventilation holes 58 formed between the dies and has molding grooves 57 for the reinforcing member 33 formed therein.

In the aforesaid jig 60, grooves 61 communicating with the aforesaid ventilation holes 58 are formed and ventilation holes 62 communicating with these grooves 61 and the vacuum pump 63 are formed. The ventilation holes 62 are connected to the vacuum pump 63 via an exhaust pipe 64.

In the present invention, how the connection is established from the ventilation holes 58 of the molding dies 56 to the vacuum pump 63 is not limited to that shown in Fig. 16 and Fig. 17, and in a word, any way of connection is adopted as long as it enables the molding dies 56 to mold the coil-shaped indentation by having the molding grooves 57 and vacuum pump 63 communicate with each other to absorb the constituting material of the reinforcing member 33 into said shape.

Next, an example will be given about a method of producing the medical tube 31 with the bellows-shaped reinforcing member of the present invention mounted thereon by the above-described production device.

The constituting material of the tube 32 is extruded from the extruding device 52 while the constituting material of the reinforcing member 33 is extruded from the extruding device 53, so that they meet inside the mouth part die 74.

Subsequently, they are extruded from the mouth part die 74 in the state in which the constituting material of the reinforcing member 33 covers the outer periphery of the tube 32.

While the constituting material of the tube 32 and the constituting material of the reinforcing member 33 are thus extruded at the same time, the molding dies 56 sandwich the outer peripheries thereof. The spaces between the constituting material of the reinforcing member 33 and the molding dies 56 are absorbed by the aforementioned vacuum pump 63 to bring the constituting material of the reinforcing member 33 into close contact with the molding grooves 57 of the molding dies 56 so that the coil-shaped indentation is formed in the constituting material of the reinforcing member 33 so as to have a predetermined pitch P, angle θ, and so on, thereby forming the constituting material of the reinforcing member 33 into the bellows-shape.

It is also possible to extrude the constituting material of the tube 32 and the constituting material of the reinforcing member 33 at the same time and sandwich the outer peripheries thereof by the molding dies 56 while bringing the constituting material of the reinforcing member 33 into close contact with the molding grooves 57 of the molding dies 56 and introducing an air pressure from inside of the tube 32 in order to facilitate the forming of the coil-shaped indentation.

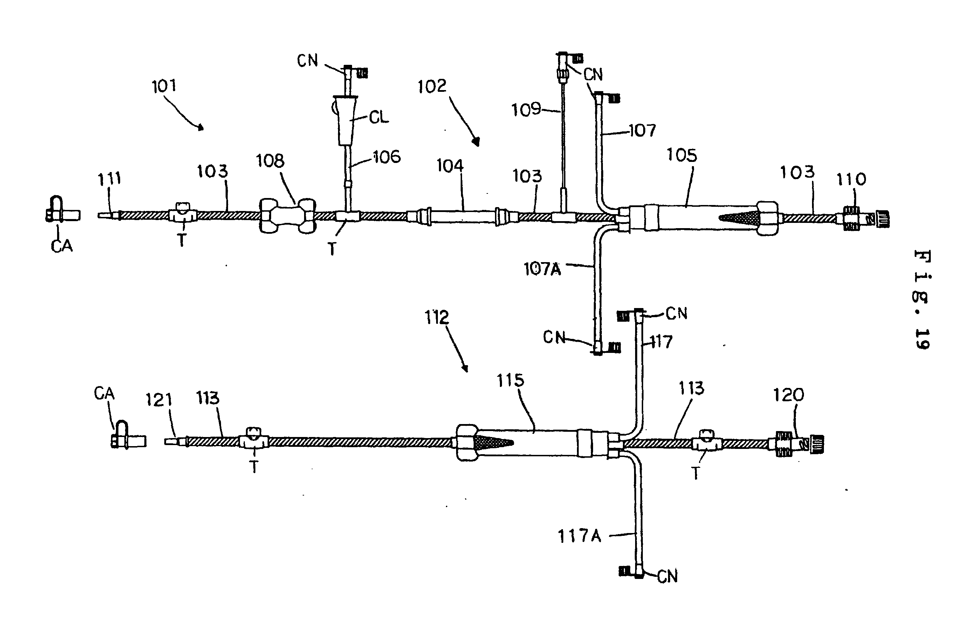

The medical tube of the present invention is preferably used as a constituting member of a medical appliance (a blood circuit) 101 as shown in Fig. 19.

The medical appliance (blood circuit) 101 is composed of artery-side main tubes 103 constituting an artery-side circuit 102 and vein-side main tubes 113 constituting a vein-side circuit 112, and in the present invention, at least a part of each is formed by the medical tube in which the coil-shaped reinforcing member is mounted on the outer periphery of the tube. Specifically, this reinforcing member may be the mono-filament, the small-diameter tubular body, or the bellows-shaped reinforcing member having the coil-shaped indentation.

In the artery-side circuit 102, a shunt connector 111, a connecting pipe T (a mix-injection portion with a plug mounted thereon for needle to insert), a negative pressure detector 108, a connecting pipe T (T-pipe) to which an infusion tube 106 for washings such as physiological saline solution is connected, a rolling tube 104, a connecting pipe T (T-pipe) to which a heparin infusion tube 109 is connected, a drip chamber 105 to which a pressure monitor tube 107 and a supplemental fluid tube 107A are connected, and a blood processing connector 110 are arranged from an upstream side (a shunt connector 111 side) toward a downstream side (a blood processing connector 110 side), and they are connected via the artery-side main tubes 103 respectively.

In the vein-side circuit 112, a blood processing connector 120, a connecting pipe T (a mix-injection portion), a drip chamber 115 to which a pressure monitor tube 117 and a supplemental fluid tube 117A are connected, a connecting pipe T (a mix-injection portion), and a shunt connector 121 are arranged from an upstream side (a blood processing connector 120 side) toward a downstream side (a shunt connector 121 side), and they are connected via the vein-side main tubes 113 respectively. In the drawing, CN signifies a connector, CA signifies a cap, and CL signifies a clamp.

The medical appliance (blood circuit) in the present invention can realize the reduction in the total weight when the artery-side main tubes 103 constituting the artery-side circuit 2 and the vein-side main tubes 113 constituting the vein-side circuit 112 are formed by the medical tubes in which the respective reinforcing members are mounted on the outer peripheries of the tubes.

Moreover, this medical appliance (blood circuit) can realize further reduction in the total weight when the washings infusion tube 106, the pressure monitor tubes 107, 117, the supplemental fluid tubes 107A, 117A, the drip chambers 105, 115 (only extrusion tubes) are also formed by the aforesaid medical tube.

To be concrete, the medical tube of the present invention with the reinforcing member mounted thereon (especially in the case of the medical tube produced by using the device in Fig. 16 to Fig. 18) can be used in the following manner when it is used in the medical appliance (blood circuit) 101 as shown in Fig. 19.

In the artery-side circuit 102, the main tube 103 in which at least one place or more among the following is(are) continuously molded is usable:
(3A) from the main tube 103 on the shunt connector 111 side to the main tube 103 extending to an end portion of the rolling tube 104 via the negative pressure detector 108;
(3B) from the main tube 103 at the other end portion of the rolling tube 104 to the main tube 103 extending to an end portion of the drip chamber 105; and
(3C) from the main tube 103 at the other end portion of the drip chamber 105 to the main tube 103 extending to the blood processing connector 110 side.

Meanwhile, in the vein-side circuit 112, the main tube 113 in which at least one place or more among the following is(are) continuously molded is usable:
(13A) from the main tube 113 on the upstream side (the blood processing connector 120 side) to the main tube 113 extending to an end portion of the drip chamber 115; and
(13B) from the main tube 113 at the other end portion of the drip chamber 115 to the main tube 113 extending to the shunt connector 121 side.

As described above, the medical tube of the present invention with the reinforcing member mounted thereon is used in the medical appliance (blood circuit) 101 as shown in Fig. 19, thereby bringing about the advantageous effect that blood disposition which has been sometimes caused in the corners of the negative pressure detector 108 and the drip chambers 105, 115 can be eliminated.

Hereinafter, embodiments of the present invention will be shown as examples.

### (Example 1)

The tube 2 as shown in Fig. 2 was used, and such setting was made that the thickness TW thereof was 0.4 mm, the diameter CD of the reinforcing member 3 constituted by the mono-filament was 0.8 mm, the number of the coils NC of the reinforcing member 3 on the outer periphery of this tube 2 was 4, the pitch P was 3.0 mm, and the lead L was 12.0 mm, thereby obtaining the medical tube 1. When this medical tube was used in the main tubes 103, 113 and so on of the blood circuit line 101 as shown in Fig. 19, reduction in weight by approximately 50% was realized compared with the case when the conventional tube 12A (the thickness TW' is 1.0 to 1.3 mm) was used. The total weight of the entire blood circuit line could be also reduced by 30%. The result will be shown in Table 1.

**Table 1**

| Name of component | Blood circuit line using medical tube 1 of present invention | Blood circuit line using conventional tube 12A | Reduction rate of weight (%) |
|---|---|---|---|
| Main tubes 103, 113 | 63.9 g | 124.6 g | 48.7% |
| Pressure monitor tubes 107, 117 and supplemental fluid tubes 107A, 117A | 21.2 | 32.6 | 35.0 |
| Chamber tubes (drip chambers 105, 115) | 14.9 | 31.0 | 51.9 |
| Other extrusion molded molded components | 45.6 | 45.6 | - |
| Injection molded components (110, 120, 111, 121, CN, CL, CA, T) | 52.7 | 52.7 | - |
| Total | 198.3 | 286.5 | 30.8 |
| Note 1) The numerals and symbols in the parenthesis of injection molded components correspond to the respective components in Fig. 19. The calculation was made on the assumption that the weight is not changed while the shape is changed. | | | |
| Note 2) Other extrusion molded components consist of the rolling tube 104, the heparin infusion tube 109, the negative pressure detector 108, and a body for infusion solution (the washings infusion tube 106). | | | |

### (Example 2)

Similarly to the Example 1, the tube 2 as shown in Fig. 2 was used, and such setting was made that the thickness TW thereof was 0.3 mm, the diameter CD of the reinforcing member 3 constituted by the mono-filament was 0.5 mm, the number of the coils NC of the reinforcing member 3 on the outer periphery of the tube 2 was 4, the pitch P was 3.0 mm, and the lead L was 12.0 mm, thereby obtaining the medical tube 1. When this medical tube 1 was used in the main tubes 103, 113 and so on of the blood circuit line 101 shown in Fig. 19, reduction in weight by approximately 65% was realized compared with the case when the conventional tube 12A (the thickness TW' is 1.0 to 1.3 mm) was used. The total weight of the entire blood circuit line could be also reduced by 37%. The result will be shown in Table 2.

**Table 2**

| Name of component | Blood circuit line using medical tube 1 of present invention | Blood circuit line using conventional tube 12A | Reduction rate of weight (%) |
|---|---|---|---|
| Main tubes 103, 113 | 50.0 g | 124.6 g | 60.0% |
| Pressure monitor tubes 107, 117 and supplemental fluid tubes 107A, 117A | 19.0 | 32.6 | 42.0 |
| Chamber tubes (drip chambers 105, 115) | 14.0 | 31.0 | 55.0 |
| Other extrusion molded components | 45.6 | 45.6 | - |
| Injection molded components (110, 120, 111, 121, CN, CL, CA, T) | 52.7 | 52.7 | - |
| Total | 181.3 | 286.5 | 37.0 |
| Note 1) The numerals and symbols in the parenthesis of injection molded components correspond to the respective components in Fig. 19. The calculation was made on the assumption that the weight is not changed while the shape is changed. | | | |
| Note 2) Other extrusion molded components consist of the rolling tube 104, the heparin infusion tube 109, the negative pressure detector 108, and a body for infusion solution (the washings infusion tube 106). | | | |

As described above, in the blood circuit line provided with the artery-side circuit and the vein-side circuit, at least a part of the artery-side main tubes constituting the artery-side circuit and a part of the vein-side main tubes constituting the vein-side circuit are formed by the medical tubes each having the reinforcing member mounted thereon, and these medical tubes are connected to each other via the connecting pipe by at least one means selected from the group consisting of (A) machining, (B) adhesive, and (C) insert molding, or by the combination of these means.

In the medical tube of the present invention, an end portion thereof can be processed in the following manner to enable the medical tube to be solvent-bonded to other constituting members (for example, connecting pipes for tubes such as a T-pipe, a Y-pipe, and a large-diameter pipe) of a medical appliance such as a blood circuit, a transfusion/infusion set, an infusion bag, or a blood bag.

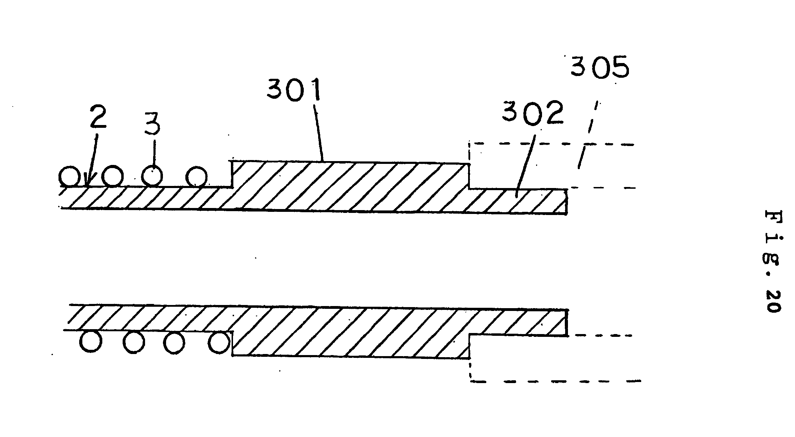

For example, as shown in Fig. 20, connecting tubes 301, 302 are mounted (including welding, connection, and integral molding) on one end portion of the tube with the coil-shaped reinforcing member 3 mounted thereon (hereinafter, abbreviated to a "tube 2 with the coil"), and a solvent is applied on the outer periphery of the connecting tube 302, which can be connected to the inner surface of a connecting pipe 305.

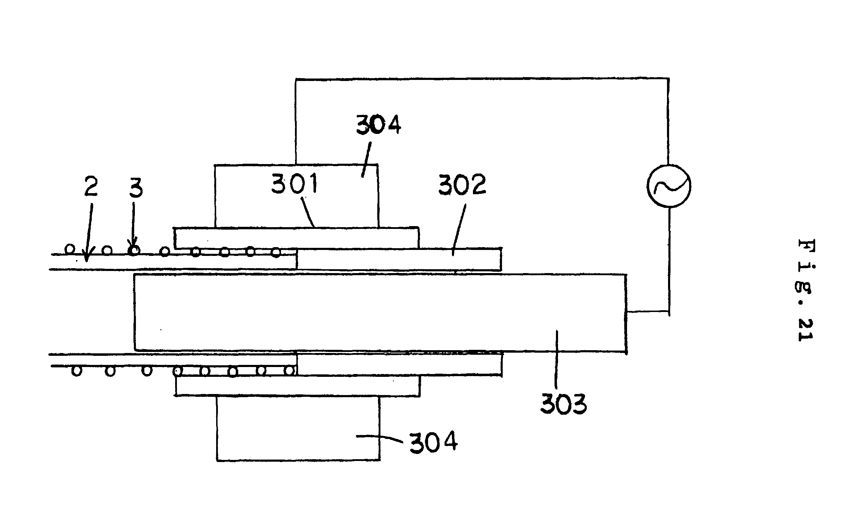

Alternatively, as shown in Fig. 21, the large-diameter connecting tube 301 is mounted on the outer periphery of the end portion of the tube 2 with the coil (made of polyvinyl chloride), the small-diameter connecting tube 302 is mounted on the inner side of the large-diameter connecting tube 301, and the end portion of the tube 2 with the coil and an end portion of the small-diameter connecting tube 302 are brought into contact with each other. in this state, a cored bar 303 is inserted from the other end portion of the small-diameter connecting tube 302 along the inner side of the tube 2 with the coil, and the tubes 2, 301, 302 are sandwiched by upper and lower high-frequency welding dies 304, 304 to apply high frequency thereto. In this way, the end portion of the tube 2 and the end portion of the tube 302, and the inner side of the tube 301 and the respective outer sides of the tubes 2, 302 are weld-connected (the coil-shaped reinforcing member 3 on the outer periphery of the tube 2 is melted at the same time).

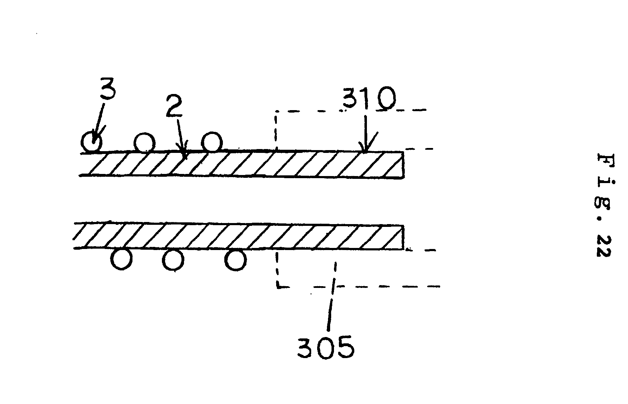

Alternatively, as shown in Fig. 22, the outer periphery of the end portion of the tube 2 with the coil is flatly processed to form a flat portion 310 and a solvent is applied on this flat portion 310, which can be connected to the inner surface of the connecting pipe 305.

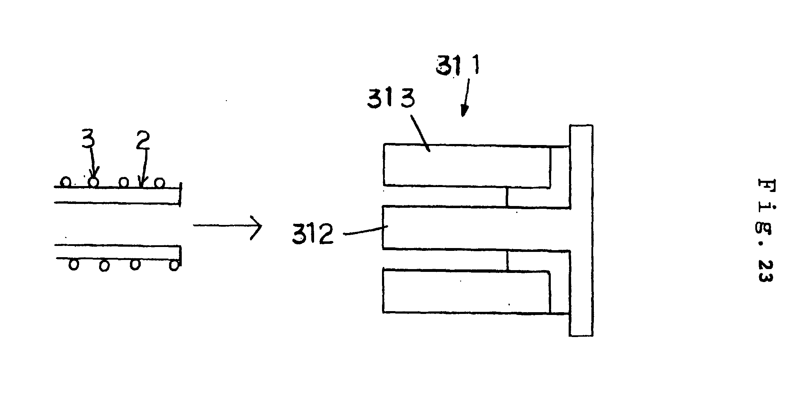

As for the formation of such a flat portion, the outer periphery of the end portion of the tube 2 with the coil can be processed to be flat in such a manner, for example, that the end portion of the tube 2 with the coil is inserted into a space formed between an inner die 312 and an outer die 313 of a molding die 311 consisting of the inner die 312 and the outer die 313 as shown in Fig. 23 and high frequency is applied thereto. Incidentally, the high-frequency applied to the aforesaid high-frequency welding dies 304 and the molding die 311 is replaceable by other means such as ultrasonic wave or heating according to the constituting material of the tube 2 with the coil.

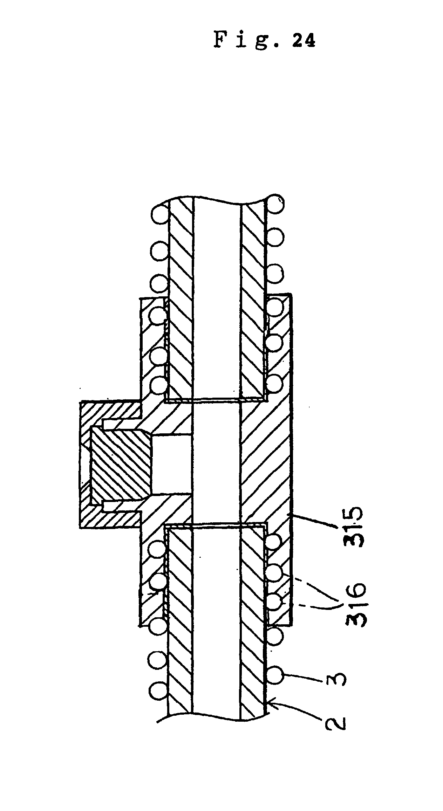

Alternatively, for example, as shown in Fig. 24, a thread groove 316 is formed on an inner surface of an end potion of a connecting pipe 315 so as to match the shape of the outer periphery of the end portion of the tube 2 with the coil (the pitch P, the lead L, the winding angle θ of the groove are made to match those of the tube 2 with the coil), and a solvent is applied on the outer periphery of the end portion of the tube 2 with the coil, thereby enabling the tube 2 with the coil to be connected to the thread grooves 316.

The medical tube of the present invention, when used as the constituting member of the main tubes or the like of the medical appliance such as the blood circuit, is preferably formed as a medical tube member by mounting a connecting pipe on an end portion of this tube. Hereinafter, specific forms of constituting the medical tube members will be explained.

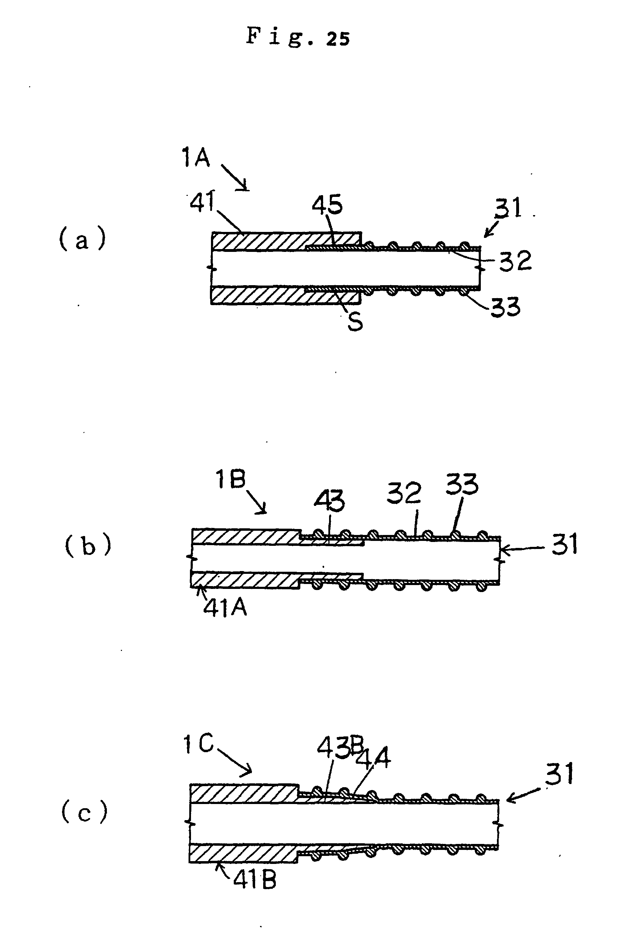

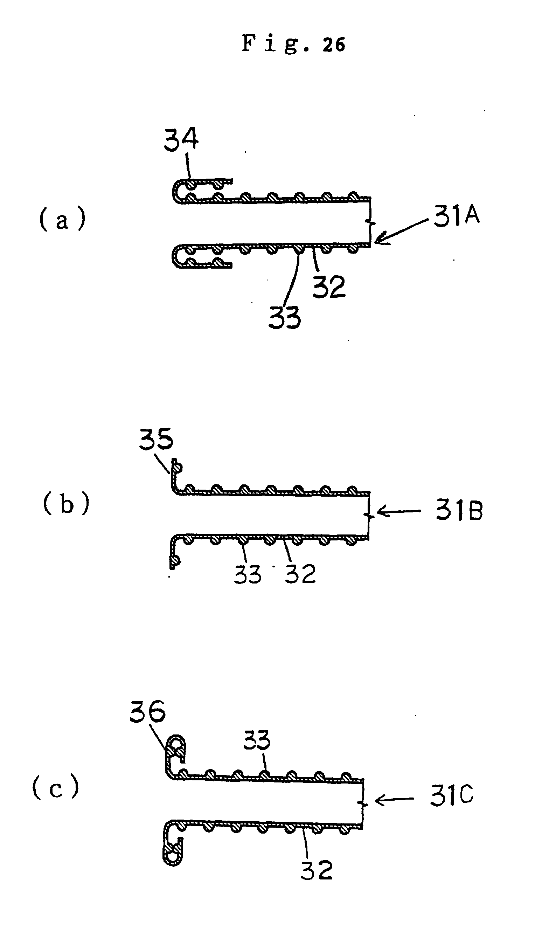

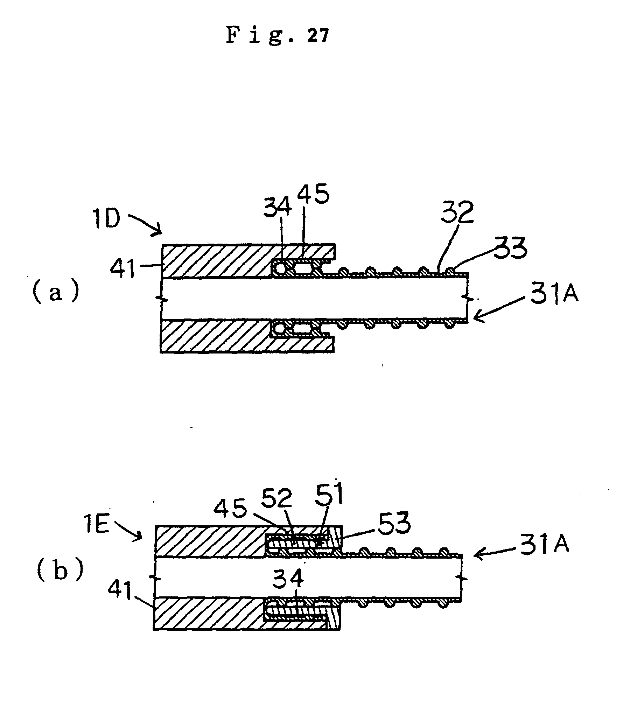

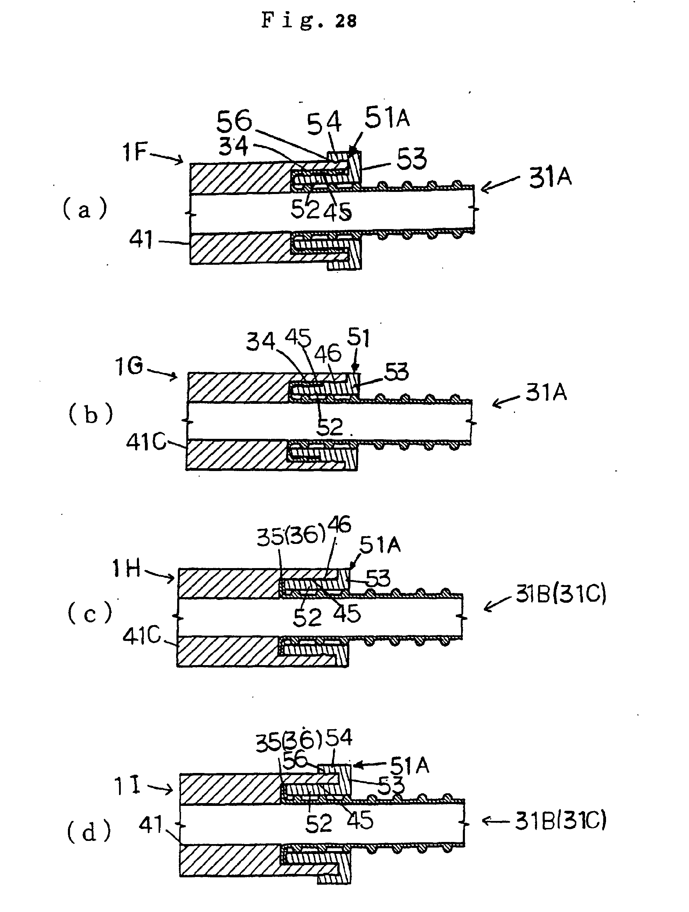

Fig. 25 is a schematic explanatory view showing examples of how the medical tube of the present invention with the reinforcing member mounted thereon is connected to a connecting pipe, Fig. 26 is a schematic explanatory view showing examples of tip processing of the medical tube of the present invention, and Fig. 27 and Fig. 28 are schematic explanatory views showing examples of how the medical tube of the present invention and a connecting pipe C are connected to each other.

As in a medical tube member 1A, for example, shown in Fig. 25(a), a tip of a tube 31 with a reinforcing member mounted thereon is formed to be a connecting portion S having a flat outer periphery, for example, by integrally welding the outer periphery of the tube 32 and the coil-shaped reinforcing member 33 so that this connecting portion S can be mounted on the groove portion 45 of the connecting pipe 41 (this has been already explained conceptually in Fig. 22).

In this embodiment, when the constituting materials of the tube 31 with the reinforcing member and the connecting pipe 41 are both polyvinyl-chloride-based materials mentioned above, a. solvent bonding, b. high-frequency welding, or e. bonding by an ultraviolet curing agent is adoptable.

When the constituting materials of the tube 31 with the reinforcing member mounted thereon and the connecting pipe 41 are both polyolefin-based materials mentioned above, c. hot-melt welding, d. ultrasonic welding, or e. bonding by an ultraviolet curing agent is adoptable.

Alternatively, as in a medical tube member 1B in Fig. 25(b), the tube 31 is pressure-inserted onto the outer periphery of a small-diameter portion 43 of a connecting pipe 41A so that the tube 31 can be mounted on the connecting pipe 41A. In this form, since a step(flat edge) is caused between the tube 31 with the reinforcing member and the small diameter portion 43, the shape of step of the small-diameter potion 43 can be improved as in Fig. 25(c).

In this embodiment, when the constituting materials of the tube 31 with the reinforcing member mounted thereon and the connecting pipe 41A are both polyvinyl-chloride-based materials mentioned above, a. solvent bonding, b. high-frequency welding, or e. bonding by an ultraviolet curing agent is adoptable.

When the constituting materials of the tube 31 and the connecting pipe 41A are both polyolefin-based materials mentioned above, c. hot-melt welding, d. ultrasonic welding, or e. bonding by an ultraviolet curing agent is adoptable.

Alternatively, as in a medical tube member 1C in Fig. 25(c), the outer periphery of a small diameter portion 43B is formed to be a taper 44 (it may be a taper becoming narrower toward a tip thereof, only the tip may be formed to be tapered, or it may be a two-tiered or a more than two-tiered taper), and the tube 31, after being heated for softening, can be pressure inserted and mounted onto the outer periphery of the small-diameter portion 43B, in which the taper 44 is formed, of a connecting pipe 41B. In this connection form, the liquid flow can be made smooth compared with that in Fig. 25(a) since no step is produced between the small-diameter portion 43B and the tube 31 with the reinforcing member.

In this embodiment, when the constituting materials of the tube 31 with the reinforcing member mounted thereon and the connecting pipe 41B are both polyvinyl-chloride-based materials mentioned above, a. solvent bonding, b. high-frequency welding, or e. bonding by an ultraviolet curing agent is adoptable.

When this constituting materials of the tube 31 and the connecting pipe 41B are both polyolefin-based materials mentioned above, c. hot-melt welding, d. ultrasonic welding, or e. bonding by an ultraviolet curing agent is adoptable.

Alternatively, as shown in Fig. 26, a tube 31A, 31B, or 31C with reinforcing member mounted thereon can be connected to the connecting pipe 41 in which the groove portion 45 is formed by forming turn-up portion 34, 35, or 36 at a tip thereof.

For example, as shown in Fig. 26(a), the outer tubular turn-up portion 34 is formed while a tip of the tube 31A with the reinforcing member mounted thereon is in contact with a heated die (not shown) or the like to be softened by heating.

Alternatively, for example, as shown in Fig. 26 (b, c), the flange-shaped turn-up portion 35 or 36 can be also formed while a tip of the tube 31B or 31C is in contact with the heated die or the like to be softened by heating. Here, the turn-up portion 35 in Fig.26 (b) is a single flange while the turn-up portion 36 in Fig.26 (c) is a two-layered flange.

Alternatively, connection methods as in Fig. 27 can be also adopted. In a medical tube member 1D in Fig. 27(a), the outer periphery of the turn-up portion 34 of the tube 31A with the reinforcing member mounted thereon can be mounted into the groove portion 45 of the connecting pipe 41.

In this embodiment, when the constituting materials of the tube 31A with the reinforcing member mounted thereon and the connecting pipe 41 are both polyvinyl-chloride-based materials mentioned above, a. solvent bonding, b. high-frequency welding, or e. bonding by an ultraviolet curing agent is adoptable.

When the constituting materials of the tube 31A and the connecting pipe 41 are both polyolefin-based materials mentioned above, c. thermal welding, d. ultrasonic welding, or e. bonding by an ultraviolet curing agent is adoptable.

Alternatively, as in a medical tube member 1E in Fig. 27(b), the outer periphery of the turn-up portion 34 of the tube 31A with the reinforcing member mounted thereon is mounted into the groove portion 45 of the connecting pipe 41, and a caulking ring 51 (having a flange portion 53 formed at an end portion of a body portion 52) is pressure-inserted into a space between a forward portion of the tube 31 with the reinforcing member and the turn-up portion 34, so that the turn-up portion 34 is sandwiched by the body portion 52 of the caulking ring 51 and the connecting pipe 41, thereby enabling the tube 31A with the reinforcing member to be mounted on the connecting pipe 41.

In this embodiment form, when the constituting materials of the tube 31A with the reinforcing member mounted thereon and the connecting pipe 41 are both polyvinyl-chloride-based materials mentioned above, a combined means among a. solvent bonding, b. high-frequency welding, and e. bonding by an ultraviolet curing agent is adoptable.

When the constituting materials of the tube 31A and the connecting pipe 41 are both polyolefin-based materials mentioned above, a combined means among c. hot-melt welding, d. ultrasonic welding, and c. bonding by an ultraviolet curing agent is adoptable.

Alternatively, in Fig. 28, when the turn-up portion 34, 35, or 36 of the tube 31A, 31B, or 31C with the reinforcing member mounted thereon is connected to a connecting pipe 41 or 41C in which the groove portion 45 is formed, using a caulking ring 51 or 51A (having the flange portion 53 formed at the end portion of the body portion 52, and having an outer tube 54 formed in the flange portion 53), an undercut (also called a protruding portion, or a projection) 56 or 46 is formed on either one of the inner periphery of the outer tube 54 of the caulking ring 51A and the groove portion 45 of the connecting pipe 41 or 41C, so that the tube 31A, 31B, or 31C with the reinforcing member can be connected to the connecting pipe 41 or 41C.

For example, as in a medical tube member 1F in Fig. 28(a), the outer periphery of the turn-up portion 34 of the tube 31A with the reinforcing member mounted thereon is mounted into the groove portion 45 of the connecting pipe 41, the body portion 52 of the caulking ring 51A having the outer tube 54 in which the undercut 56 is formed is pressure-inserted into a space between a forward portion of the tube 31A and the turn-up portion 34, and the turn-up portion 34 and a forward portion of the connecting pipe 41 are sandwiched between the outer tube 54 and the body portion 52, so that the tube 31A with the reinforcing member can be mounted on the connecting pipe 41. The outer periphery of the connecting pipe 41 is firmly fixed by the undercut 56.

In this embodiment, when the constituting materials of the tube 31A with the reinforcing member mounted thereon and the connecting pipe 41 are both polyvinyl-chloride-based materials mentioned above, a combined means among a. solvent bonding, b. high-frequency welding, and e. bonding by an ultraviolet curing agent is adoptable.

When the constituting materials of the tube 31A with the reinforcing member mounted thereon and the connecting pipe 41 are both polyolefin-based materials mentioned above, a combined means among c. hot-melt welding, d. ultrasonic welding, and e. bonding by an ultraviolet curing agent is adoptable.

As in a medical tube member 1G in Fig. 28(b), the undercut 46 is formed in the groove portion 45 of the connecting pipe 41C, the outer periphery of the turn-up portion 34 of the tube 31A with the reinforcing member mounted thereon is mounted into the groove portion 45 of the connecting pipe 41C, and the caulking ring 51 (having the flange portion 53 formed at the end portion of the body portion 52) is pressure-inserted into the space between the forward portion of the tube 31A and the turn-up portion 34, thereby causing the turn-up portion 34 to be sandwiched by the connecting pipe 41C and the caulking ring 51 so that the tube 31A can be mounted on the connecting pipe 41C. The outer periphery of the body portion 52 of the caulking ring 51 is firmly fixed by the undercut 46.

In this embodiment, when the constituting materials of the tube 31A with the reinforcing member mounted thereon and the connecting pipe 41C are both polyvinyl-chloride-based materials mentioned above, a combined means among a. solvent bonding, b. high-frequency welding, and e. bonding by an ultraviolet curing agent is adoptable.

When the constituting materials of the tube 31A with the reinforcing member mounted thereon and the connecting pipe 41C are both polyolefin-based materials mentioned above, a combined means among c. hot-melt welding, d. ultrasonic welding, and e. bonding by an ultraviolet curing agent is adoptable.

Alternatively, as in a medical tube member 1H in Fig. 28(c), the undercut 46 is formed in the groove portion 45 of the connecting pipe 41C, the flange-shaped turn-up portion 35 or 36 of the tube 31B or 31C with the reinforcing member mounted thereon is mounted into the groove portion 45, and the caulking ring 51A is pressure-inserted into a space surrounded by a forward portion of the tube 31B or 31C with the reinforcing member, the turn-up portion 35 or 36, and the groove portion 45, thereby causing the turn-up portion 35 or 36 to be sandwiched between the caulking ring 51A and the connecting pipe 41C, so that the tube 31B or 31C with the reinforcing member can be mounted on the connecting pipe 41C. The outer periphery of the body portion 52 of the caulking ring 51A is firmly fixed by the undercut 46.

In this embodiment form, when the constituting materials of the tubes 31B, 31C with the reinforcing members mounted thereon and the connecting pipe 41C are both polyvinyl-chloride-based materials mentioned above, a combined means among a. solvent bonding, b. high-frequency welding, and e. bonding by an ultraviolet curing agent is adoptable.

When the constituting materials of the tubes 31B, 31C with the reinforcing members mounted thereon and the connecting pipe 41C are both polyolefin-based materials mentioned above, a combined means among c. hot-melt welding, d. ultrasonic welding, and e. bonding by an ultraviolet curing agent is adoptable.

Alternatively, as in a medical tube member 1I in Fig. 28(d), the flange-shaped turn-up portion 35 or 36 of the tube 31B or 31C with the reinforcing member mounted thereon is mounted into the groove portion 45 of the connecting pipe 41, the caulking ring 51A (having the flange portion 53 formed at the end portion of the body portion 52, and further, the outer tube 54 formed in the flange portion 53) having the outer tube 54 in which the undercut 56 is formed is pressure-inserted into a space surrounded by the forward portion of the tube 31B or 31C, the turn-up portion 35 or 36, and the groove portion 45, thereby causing the turn-up portion 35 or 36 to be sandwiched between the caulking ring 51A and the connecting pipe 41, so that the tube 31B or 31C can be mounted on the connecting pipe 41.

The outer periphery of the connecting pipe 41 is firmly fixed by the undercut 56 of the outer tube 54.

In this embodiment form, when the constituting materials of the tubes 31B, 31C each having the reinforcing member mounted thereon and the connecting pipe 41 are both polyvinyl chloride based mentioned above, a combined means among a. solvent bonding, b. high-frequency welding, and e. bonding by an ultraviolet curing agent is adoptable.

When the constituting materials of the tubes 31B, 31C with the reinforcing members mounted thereon and the connecting pipe 41 are both polyolefin based mentioned above, a combined means among c. hot-melt welding, d. ultrasonic welding, and e. bonding by an ultraviolet curing agent is adoptable.

In the present invention, instead of "forming the undercut (also called a protruding portion, or a projection) 56 or 46 on either one of the inner periphery of the outer tube 54 of the caulking ring 51A and the groove portion 45 of the connecting pipe 41C to connect the tube 31A, 31B, or 31C with the reinforcing member and the connecting pipe 41 or 41C" as described above, it is also preferable that projections and grooves are formed on wall surfaces facing each other of the aforesaid caulking ring 51 or 51A and the aforesaid connecting pipe 41 or 41C respectively (in the caulking ring 51, the body portion 52 and the outer tube 54, and in the connecting pipe 41, the groove portion 45, which is in contact with the aforesaid body portion 52 and the outer tube 54, and the outer periphery) to connect them to each other by engagement or fitting. Alternatively, it is also preferable that the respective protrusions and grooves facing each other described above are formed in a screw-shaped to connect the tube 31A, 31B, or 31C with the reinforcing member and the connecting pipe 41 or 41C by screwing.

In the present invention, in order to "connect (mount)" the medical tube with the reinforcing member mounted thereon to (on) other constituting member (for example, a connecting pipe for a tube and so on such as a T-pipe, Y-pipe, and a large-diameter pipe, which will be hereinafter abbreviated to a connecting pipe) of the medical appliance, the following are adoptable according to the constituting materials of both the medical tube and the connecting pipe and according to the type of connection, as is described above:
(A) machining; (B) adhesive; (C) insert molding; and so on.

For example, the following connecting means or the combination of these connecting means are included: (a) solvent bonding (used for connecting, for example, polyvinyl chloride to each other); (b) high-frequency welding (used for connecting, for example, polyvinyl chloride to each other, and ethylene vinyl acetate copolymers to each other); (c) hot-melt welding (used for connecting, for example, polyethylene to each other, polypropylene to each other, and polyethylene and polypropylene each other); (d) ultrasonic welding (used for connecting, for example, polyethylene to each other, polypropylene to each other, and polyethylene and polypropylene each other); (e) bonding by an ultraviolet curing agent (means of applying an ultraviolet curing agent between the tube and the connecting pipe to irradiate an ultraviolet ray for curing); (f) fitting by caulking (including a means of pressure-inserting the caulking ring between the tube and the connecting pipe, a means of covering the outer peripheries of the tube 1 and the connecting pipe with the large-diameter tube or a shrinkable tube, and the like. Further, the undercut (also called a protruding portion) may be formed in the caulking ring or the connecting pipe to fix the connecting pipe or the caulking ring, or the protrusions and the grooves facing each other are formed in the caulking ring and the connecting pipe respectively to fix them by engagement or fitting. Further, the aforesaid protrusions and grooves facing each other may be formed in the screw shape to fix them by screwing.); (g) engagement (a means of forming protrusions and recesses for mutual engagement both in the tube and the connecting pipe and engaging them with each other, or when necessary, gel can be inserted between the protrusions and recesses); (h) laser beam; (i) close-contact bonding (used for connecting, for example, polyethylene to each other, polypropylene to each other, and polyethylene and polypropylene each other), and so on.

In the above description, (A) machining includes (b) high-frequency welding, (c) hot-melt welding, (d) ultrasonic welding, (f) fitting by caulking, (g) engagement, and (h) laser beam, which are mentioned above.

In the above description, (B) adhesive includes (a) solvent boning, (e) bonding by an ultraviolet curing agent, and (i) close-contact bonding, which are mentioned above.

In the above description, (C) insert molding is a means of disposing the medical tube inside a die and covering the outer periphery thereof with the constituting member of the connecting pipe 41.

When the constituting materials of the tube and the connecting pipe are both polyvinyl-chloride-based materials (including chlorinated polyethylene) (also including the case when these materials are used for a part of a connecting layer), (a) solvent welding, (b) high-frequency welding, (e) bonding by an ultraviolet curing agent, (f) fitting by caulking, (g) engagement, or the like, which are mentioned above, or the combination of these connecting means is adoptable.

When the constituting materials of the tube and the connecting pipe are both, such materials other than the aforesaid polyvinyl-chloride-based materials, for example, polyolefin-based materials (polyethylene-based materials, polypropylene-based materials, copolymers including a monomer copolymerizable with them, or the like) (including the case when these materials are used for a part of the connecting layer) (c) hot-melt welding, (d) ultrasonic welding, (e) bonding by an ultraviolet curing agent, (f) fitting by caulking, (g) engagement, (i) close-contact bonding, or the combination of these connecting means is adoptable.

Incidentally, examples of the preferred combination of the materials of the tube and the reinforcing member mounted on the surface of this tube for use in the present invention are, though not specially limited thereto, as follows:

When the constituting material used for the medical tube with the reinforcing member mounted thereon of the present invention is polyvinyl chloride, nonrigid(flexible) polyvinyl chloride with a large compounding rate (exceeding 30 mass % to 80 mass %) of a plasticizer is used for the constituting material of the tube, and rigid polyvinyl chloride with a low compounding rate (from 10 mass % to 30 mass %) of the plasticizer is used for the constituting material of the coil-shaped reinforcing member.

When a material other than polyvinyl chloride, for example, polyolefin, is used and this material consists of a single substance (for example, polyethylene), the substance property (density) of this material is selected so that, for example, nonrigid(flexible)low-density polyethylene (density: 0.910) can be used for the tube and relatively rigid high-density polyethylene (high density: 0.930) can be used for the coil-shaped reinforcing member.

When this material consists of two substances or more (for example, a copolymer of ethylene and vinyl acetate, a random copolymer of ethylene and methyl acrylate, or the like), the crystallinity (degree of polymerization) of the material is selected so that, for example, a copolymer of nonrigid ethylene with low-crystallinity (low degree of polymerization) and vinyl acetate (methyl acrylate) can be used for the tube and a copolymer of relatively rigid ethylene with relatively high crystallinity (high degree of polymerization) and vinyl acetate (methyl acrylate) can be used for the coil-shaped reinforcing member. Generally, ethylene, when used as a single substance, has a high crystallinity and regularly-crystallized ethylene has a high hardness. On the other hand, when a monomer (vinyl acetate, methyl acrylate) copolymerizable with ethylene is added thereto, the hardness of the whole copolymer is lowered since the growth of an ethylene crystal is restrained as the added amount (degree of polymerization) is increased.

Alternatively, a copolymer of nonrigid ethylene with low-crystallinity (low degree of polymerization) and vinyl acetate (methyl acrylate) can be used for the tube and relatively rigid polyethylene with high density (high density: 0.930) can be used for the coil-shaped reinforcing member.

### Industrial Utility

The following effects are provided by the use of a medical tube of the present invention in which a coil-shaped reinforcing member is mounted on the outer periphery of a tube.
(1) The total weight of the tube is so reduced that labor saving in the packaging and sterilizing processes thereof is realized, and package volume thereof is so downsized that cost of packaging materials, transportation, and so on can also be reduced.
(2) Functions such as anti-kinking property, antiblocking property, and so on can be enhanced in spite of the weight reduction.
(3) The amount of the materials used can be saved to a half or smaller so that effective utilization of resources can be realized. The amount of wastes can be also reduced by half or less, which contributes to environmental protection.
(4) The tube can sufficiently resist the pressure increase inside the tube.

## Claims

1. A medical tube, wherein a coil-shaped reinforcing member is mounted on the outer periphery of said tube.

2. The medical tube according to claim 1, wherein said reinforcing member is a mono-filament or a small-diameter tubular body.

3. The medical tube according to claim 1, wherein said reinforcing member is a bellows-shaped reinforcing member having a coil-shaped concave-convex.

4. The medical tube according to claim 1 or 2, wherein said coil-shaped reinforcing member is so formed that the pitch P is 2.0 to 5.0 mm.

5. The medical tube according to claim 1, 2 or 4,
wherein said coil-shaped reinforcing member is so formed that the number of coils NC on the outer periphery of said tube is 1 to 10.

6. The medical tube according to claim 1, 2, 4, or 5,
wherein said coil-shaped reinforcing member is so formed that the lead L on the outer periphery of said tube is 2.0 to 50.0 mm.

7. The medical tube according to any one of claims 1, 2, and 4 to 6, wherein said coil-shaped reinforcing member is so formed that the winding angle θ relative to a center line C of said tube in a longitudinal direction is 60 degrees to 85 degrees.

8. The medical tube according to any one of claims 1, 2 and 4 to 7, wherein the area S that the coil-shaped reinforcing member covering the surface of said tube occupies is 30 % or larger.

9. The medical tube according to claim 3, wherein said bellows-shaped reinforcing member is so formed that the pitch P of a protruding portion T is 1.0 to 5.0 mm.

10. The medical tube according to claim 3 or 8, wherein the angle θ made by the center line C of said tube in a longitudinal direction and said protruding portion T is 60 degrees to 90 degrees.

11. A production method for the medical tube according to any one of claims 1, 2, and 4 to 8, the production method comprising the steps of:
(1) extruding the tube from an extrusion die; and
(2) mounting the reinforcing member in the coil form on the outer periphery of the tube while said extruded tube is hot.

12. A production method for the medical tube according to any one of claims 1, 2, and 4 to 8, the production method comprising the steps of:
(1) extruding the tube from an extrusion die;
(2) extruding the reinforcing member from another extrusion die; and
(3) mounting said extruded reinforcing member in a coil form on the outer periphery of said extruded tube.

13. A production device for the medical tube according to any one of claims 1, 2, and 4 to 8, the production device comprising an extrusion molding means for the tube, a take-up means for the reinforcing member, a supply means for the reinforcing member, and a rotation applying means for the take-up means,
wherein said rotation applying means transmits rotation to said take-up means, said supply means for the reinforcing member feeds the reinforcing member, and said take-up means takes up said reinforcing member to send said taken-up reinforcing member in an extruding direction of the tube extruded from the extrusion molding means in sequence, thereby mounting said reinforcing member on the outer periphery of said tube.

14. The production device for the medical tube according to claim 13,
wherein said extruding means for the tube comprises an extrusion die for the tube composed of an outer die and an inner die,
wherein said take-up means for the reinforcing member is constituted by a take-up device,
wherein said supply means for the reinforcing member is constituted by a supply device, and
wherein said rotation applying means for the take-up means is constituted by a motor M.

15. A production device for the medical tube according to any one of claims 1, 2, and 4 to 8, the production device comprising an extrusion molding means for the tube, a take-up means for the reinforcing member, an extruding means for the reinforcing member, and a rotation applying means for the take-up means,
wherein said rotation applying means transmits rotation to said take-up means, and said extruding means for the reinforcing member extrudes the reinforcing member in the extruding direction of the tube extruded from said extrusion molding means, thereby mounting said reinforcing member on the outer periphery of said tube.

16. The production device for the medical tube according to claim 15,
wherein said extruding means for the tube comprises an extrusion die for the tube composed of an outer die and an inner die,
wherein said take-up means for the reinforcing member is constituted by a take-up device,
wherein said extruding means for the reinforcing member is constituted by an extrusion die, and
wherein said rotation applying means for the take-up means is constituted by a motor M and a belt.

17. A production method for the medical tube according to any one of claim 1, 3, 9 or 10, the production method comprising the steps of:
(1) extruding a constituting material of the tube in a tube shape;
(2) extruding a constituting material of the reinforcing member onto the outer periphery of said extruded tube;
(3) sandwiching outer peripheries of said constituting material of the tube and said constituting material of the reinforcing member by molding dies while both of the constituting materials are extruded at the same time; and
(4) absorbing spaces between said constituting material of the reinforcing member and said molding dies, bringing said constituting material of the reinforcing member into close contact with molding grooves of the molding dies, and forming the coil-shaped concave-convex in said constituting material of the reinforcing member to give it a bellows-shape.

18. A production device for the medical tube according to any one of claim 1, 3, 9, or 10, the production device comprising:
an extruding device for the tube; an extruding device for extruding the reinforcing member onto the outer periphery of the tube; and a molding device for the extruded reinforcing member.

19. The production device for the medical tube according to claim 18,
wherein said molding device for the reinforcing member comprises a molding die for the reinforcing member, a jig for said molding die, a vacuum pump, and a drive device for said molding die, and
wherein a pair of said jigs are disposed to face each other, and said molding die is movably provided on an outer periphery of each of said jigs.

20. The production device for the medical tube according to claim 19, wherein said molding die is constituted by integrating a plurality of dies, between each of which a ventilation hole is formed, and having provided a molding groove for said reinforcing member.

21. The production device for the medical tube according to claim 19, wherein said jig has grooves formed to communicate with said ventilation holes and ventilation holes formed to communicate with said grooves and said vacuum pump.

22. A medical tube, wherein a large-diameter connecting tube is mounted on the outer periphery of one end portion of the tube according to any one of claims 1 to 10 and a small-diameter connecting tube is mounted on said large-diameter connecting tube thereby enabling a connection of a connecting pipe to said small-diameter connecting tube.

23. A medical tube, wherein a flat portion is formed on the outer periphery of one end portion of the tube according to any one of claims 1 to 10 to enable a connecting pipe to be mounted on said flat portion.

24. A medical tube member, wherein a connecting pipe is mounted on one end portion of said tube according to any one of claims 1 to 10, or claim 22, or claim 23.

25. A medical tube member, wherein a connecting pipe is mounted on one end portion of said tube according to any one of claims 1 to 10, the connecting pipe having a thread groove matching the shape of the outer periphery of the end portion of said tube.

26. A medical tube member in which said tube according to any one of claims 1 to 10 and 22 to 23 and a connecting pipe 41B having provided a protruding small-diameter portion 43B are connected to each other,
wherein said tube is mounted on the outer periphery of said small-diameter portion 43 of the connecting pipe 41B.

27. A medical tube member in which said tube according to any one of claims 1 to 10 and 22 to 23 and a connecting pipe 41 having a groove portion 45 are connected to each other,
wherein an outer tubular turn-up portion 34 is formed at the tip of said tube and the outer periphery of said turn-up portion 34 is mounted on said groove portion 45 of the connecting pipe 41.

28. A medical tube member in which said tube according to any one of claims 1 to 10 and 22 to 23 and a connecting pipe 41 having a groove portion 45 are connected to each other,
wherein an outer tubular turn-up portion 34 is formed at the tip of the tube 31A on which said reinforcing member is mounted,
and the outer periphery of said turn-up portion 34 is mounted on said groove portion 45 of the connecting pipe 41, and
wherein a caulking ring 51 is pressure-inserted into a space between the forward portion of the tube 31 on which said reinforcing member is mounted and said turn-up portion 34 or into a space surrounded by said forward portion of the tube, said turn-up portion 34, and said groove portion 45.

29. A medical appliance, wherein, in a blood circuit comprising an artery-side circuit and a vein-side circuit, at least a part of an artery-side main tube constituting said artery-side circuit and a part of a vein-side main tube constituting said vein-side circuit are formed by the medical tube according to claims 1 to 10, or claim 22, or claim 23.

30. A medical appliance composed of a blood circuit comprising an artery-side circuit and a vein-side circuit, where in at least a part of an artery-side main tube constituting said artery-side circuit and a part of a vein-side main tube constituting said vein-side circuit are formed of the medical tube according to claims 1 to 10, claim 22, or claim 23, and said medical tubes are connected to each other via a connecting pipe by at least one means selected from the group consisting of (A) machining, (B) adhesive, and (C) insert molding, or a combination of these means.
